# EUROPEAN PATENT SPECIFICATION

(11) **EP 0 684 313 B1**
(45) Date of publication and mention of the grant of the patent: **05.07.2006**
(21) Application number: 95105693.6
(22) Date of filing: 15.04.1995
(51) Int. Cl.: C12N 15/55, C12N 9/16, A23K 1/165

(54) **Polypeptides with phytase activity**
Polypeptide mit phytase Wirkung
Polypeptides à activité phytase

(30) Priority: 25.04.1994 EP 94810228
(43) Date of publication of application: 29.11.1995
(73) Proprietor: DSM IP Assets B.V., 6411 TE Heerlen (NL)
(72) Inventor: Van Loon, Adolphus, CH-4310 Rheinfelden (CH); Mitchell, David, CH-4147 Aesch (CH)
(74) Representative: Schwander, Kuno

(56) References cited:
- YAMADA K ET AL: "PHYTASE FROM ASPERGILLUS TERREUS. PART I. PRODUCTION, PURIFICATION AND SOME GENERAL PROPERTIES OF THE ENZYME" AGRICULTURAL AND BIOLOGICAL CHEMISTRY, vol. 32, no. 10, July 1968, pages 1275-1282, XP002053182
- PIDDINGTON C S ET AL: "The cloning and sequencing of the genes encoding phytase (phy) and pH 2.5-optimum acid phosphatase (aph) from Aspergillus niger var awamori" GENE, vol. 133, 1993, pages 55-62, XP002089982
- MULLANEY EJ ET AL: "POSITIVE IDENTIFICATION OF A LAMBDA GT11 CLONE CONTAINING A REGION OF FUNGAL PHYTASE GENE BY IMMUNOPROBE AND SEQUENCE VERIFICATION" APPL MICROBIOL BIOTECHNOL, vol. 35, no. 5, August 1991, pages 611-614, XP002096740
- PEN J ET AL: "PHYTASE-CONTAINING TRANSGENIC SEEDS AS A NOVEL FEED ADDITIVE FOR IMPROVED PHOSPHORUS UTILIZATION" BIO/TECHNOLOGY, vol. 11, no. 7, July 1993, pages 811-814, XP002026203
- SEGUEILHA L ET AL: "PURIFICATION AND PROPERTIES OF THE PHYTASE FROM SCHWANNIOMYCES CASTELLII" JOURNAL OF FERMENTATION AND BIOENGINEERING, vol. 74, no. 1, 1992, pages 7-11, XP002053183
- MITCHELL DB ET AL: "THE PHYTASE SUBFAMILY OF HISTIDINE ACID PHOSPHATASES: ISOLATION OF GENES FOR TWO NOVEL PHYTASES FROM THE FUNGI ASPERGILLUS TERREUS AND MYCELIOPHTHORA THERMOPHILA" MICROBIOLOGY, vol. 143, 1997, pages 245-252, XP002097277

### SEQUENCE LISTING

(1) GENERAL INFORMATION:
   (i) APPLICANT:
      (A) NAME: F. HOFFMANN-LA ROCHE AG
      (B) STREET: Grenzacherstrasse 124
      (C) CITY: Basle
      (D) STATE: BS
      (E) COUNTRY: Switzerland
      (F) POSTAL CODE (ZIP): CH-4002
      (G) TELEPHONE: 061 - 688 25 05
      (H) TELEFAX: 061 - 688 13 95
      (I) TELEX: 962292/965542 hlr ch
   (ii) TITLE OF INVENTION: Polypeptides with phytase activity
   (iii) NUMBER OF SEQUENCES: 21
   (iv) COMPUTER READABLE FORM:
      (A) MEDIUM TYPE: Floppy disk
      (B) COMPUTER: Apple Macintosh
      (C) OPERATING SYSTEM: System 7.1 (Macintosh)
      (D) SOFTWARE: WOrd 5.0
   (vi) PRIOR APPLICATION DATA:
      (A) APPLICATION NUMBER: EP 94810228.0
      (B) FILING DATE: 25-APR-1994
(2) INFORMATION FOR SEQ ID NO: 1:
   (i) SEQUENCE CHARACTERISTICS:
      (A) LENGTH: 2327 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: join (374..420, 469..1819)
   (xi) SEQUENCE DESCRIPTION: SEQ ID NO: 1:
   TCTAGAACAA TAACAGGTAC TCCCTAGGTA CCCGAAGGAC CTTGTGGAAA ATGTATGGAG     60
   GTGGACACGG CACCAACCAC CACCCGCGAT GGCGCACGTG GTGCCCTAAC CCCTTGCTCC    120
   CTCAGGATGG AATCCATGTC GACTCTTTAC CCTCACCATC GCCTGGATGA AACCTCCCCG    180
   CTAAGCTCAC GACGATCGCT ATTTCCGACC GATTTGACCG TCATGGTGGA GGGCTGATTC    240
   GGTCGATGCT CCTGCCTTCA TTTCGGAGTT CGGAGACATG AAAGGCTTAT ATGAGGACGT    300
   CCCAGGTCGG GGACGAAATC CGCCCTGGGC TGTGCTCCTT CGTCGGAAAC ATCTGCTGTC    360
   CGTGATGGCT ACC ATG GGC TTT CTT GCC ATT GTG CTC TCC GTC GCC TTG         409
                  Met Gly Phe Leu Ala Ile Val Leu Ser Val Ala Leu
                   1               5                  10
   CTC TTT AGA  AG  GTATGCACCC CTCTACGTCC AATTCTCTGG GCACTGACAA           460
   Leu Phe Arg  Ser
           15
   CGGCGCAG C ACA TCG GGC ACC CCG TTG GGC CCC CGG GGC AAA CAT AGC         508
              Thr Ser Gly Thr Pro Leu Gly Pro Arg Gly Lys His Ser
                           20                  25
   GAC TGC AAC TCA GTC GAT CAC GGC TAT CAA TGC TTT CCT GAA CTC TCT        556
   Asp Cys Asn Ser Val Asp His Gly Tyr Gln Cys Phe Pro Glu Leu Ser
    30                 35                  40                  45
   CAT AAA TGG GGA CTC TAC GCG CCC TAC TTC TCC CTC CAG GAC GAG TCT        604
   His Lys Trp Gly Leu Tyr Ala Pro Tyr Phe Ser Leu Gln Asp Glu Ser
                   50                  55                  60
   CCG TTT CCT CTG GAC GTC CCA GAG GAC TGT CAC ATC ACC TTC GTG CAG        652
   Pro Phe Pro Leu Asp Val Pro Glu Asp Cys His Ile Thr Phe Val Gln
               65                  70                  75
   GTG CTG GCC CGC CAC GGC GCG CGG AGC CCA ACC CAT AGC AAG ACC AAG        700
   Val Leu Ala Arg His Gly Ala Arg Ser Pro Thr His Ser Lys Thr Lys
           80                  85                  90
   GCG TAC GCG GCG ACC ATT GCG GCC ATC CAG AAG AGT GCC ACT GCG TTT        748
   Ala Tyr Ala Ala Thr Ile Ala Ala Ile Gln Lys Ser Ala Thr Ala Phe
       95                  100                 105
   CCG GGC AAA TAC GCG TTC CTG CAG TCA TAT AAC TAC TCC TTG GAC TCT        796
   Pro Gly Lys Tyr Ala Phe Leu Gln Ser Tyr Asn Tyr Ser Leu Asp Ser
   110                 115                 120                 125
   GAG GAG CTG ACT CCC TTC GGG CGG AAC CAG CTG CGA GAT CTG GGC GCC        844
   Glu Glu Leu Thr Pro Phe Gly Arg Asn Gln Leu Arg Asp Leu Gly Ala
                   130                 135                 140
   CAG TTC TAC GAG CGC TAC AAC GCC CTC ACC CGA CAC ATC AAC CCC TTC        892
   Gln Phe Tyr Glu Arg Tyr Asn Ala Leu Thr Arg His Ile Asn Pro Phe
               145                 150                 155
   GTC CGC GCC ACC GAT GCA TCC CGC GTC CAC GAA TCC GCC GAG AAG TTC        940
   Val Arg Ala Thr Asp Ala Ser Arg Val His Glu Ser Ala Glu Lys Phe
           160                 165                 170
   GTC GAG GGC TTC CAA ACC GCT CGA CAG GAC GAT CAT CAC GCC AAT CCC        988
   Val Glu Gly Phe Gln Thr Ala Arg Gln Asp Asp His His Ala Asn Pro
       175                 180                 185
   CAC CAG CCT TCG CCT CGC GTG GAC GTG GCC ATC CCC GAA GGC AGC GCC       1036
   His Gln Pro Ser Pro Arg Val Asp Val Ala Ile Pro Glu Gly Ser Ala
   190                 195                 200                 205
   TAC AAC AAC ACG CTG GAG CAC AGC CTC TGC ACC GCC TTC GAA TCC AGC       1084
   Tyr Asn Asn Thr Leu Glu His Ser Leu Cys Thr Ala Phe Glu Ser Ser
                   210                 215                 220
   ACC GTC GGC GAC GAC GCG GTC GCC AAC TTC ACC GCC GTG TTC GCG CCG      1132
   Thr Val Gly Asp Asp Ala Val Ala Asn Phe Thr Ala Val Phe Ala Pro
               225                 230                 235
   GCG ATC GCC CAG CGC CTG GAG GCC GAT CTT CCC GGC GTG CAG CTG TCC      1180
   Ala Ile Ala Gln Arg Leu Glu Ala Asp Leu Pro Gly Val Gln Leu Ser
           240                 245                 250
   ACC GAC GAC GTG GTC AAC CTG ATG GCC ATG TGT CCG TTC GAG ACG GTC      1228
   Thr Asp Asp Val Val Asn Leu Met Ala Met Cys Pro Phe Glu Thr Val
       255                 260                 265
   AGC CTG ACC GAC GAC GCG CAC ACG CTG TCG CCG TTC TGC GAC CTC TTC      1276
   Ser Leu Thr Asp Asp Ala His Thr Leu Ser Pro Phe Cys Asp Leu Phe
   270                 275                 280                 285
   ACG GCC ACT GAG TGG ACG CAG TAC AAC TAC CTG CTC TCG CTG GAC AAG      1324
   Thr Ala Thr Glu Trp Thr Gln Tyr Asn Tyr Leu Leu Ser Leu Asp Lys
                   290                 295                 300
   TAC TAC GGC TAC GGC GGG GGC AAT CCG CTG GGT CCG GTG CAG GGG GTC      1372
   Tyr Tyr Gly Tyr Gly Gly Gly Asn Pro Leu Gly Pro Val Gln Gly Val
               305                 310                 315
   GGC TGG GCG AAC GAG CTG ATG GCG CGG CTA ACG CGC GCC CCC GTG CAC      1420
   Gly Trp Ala Asn Glu Leu Met Ala Arg Leu Thr Arg Ala Pro Val His
           320                 325                 330
   GAC CAC ACC TGC GTC AAC AAC ACC CTC GAC GCG AGT CCG GCC ACC TTC      1468
   Asp His Thr Cys Val Asn Asn Thr Leu Asp Ala Ser Pro Ala Thr Phe
       335                 340                 345
   CCG CTG AAC GCC ACC CTC TAC GCC GAC TTC TCC CAC GAC AGC AAC CTG      1516
   Pro Leu Asn Ala Thr Leu Tyr Ala Asp Phe Ser His Asp Ser Asn Leu
   350                 355                 360                 365
   GTG TCG ATC TTC TGG GCG CTG GGC CTG TAC AAC GGC ACC GCG CCG CTG      1564
   Val Ser Ile Phe Trp Ala Leu Gly Leu Tyr Asn Gly Thr Ala Pro Leu
                   370                 375                 380
   TCG CAG ACC TCC GTC GAG AGC GTC TCC CAG ACG GAC GGG TAC GCC GCC      1612
   Ser Gln Thr Ser Val Glu Ser Val Ser Gln Thr Asp Gly Tyr Ala Ala
               385                 390                 395
   GCC TGG ACG GTG CCG TTC GCC GCT CGC GCG TAC GTC GAG ATG ATG CAG      1660
   Ala Trp Thr Val Pro Phe Ala Ala Arg Ala Tyr Val Glu Met Met Gln
           400                 405                 410
   TGT CGC GCC GAG AAG GAG CCG CTG GTG CGC GTG CTG GTC AAC GAC CGG      1708
   Cys Arg Ala Glu Lys Glu Pro Leu Val Arg Val Leu Val Asn Asp Arg
       415                 420                 425
   GTC ATG CCG CTG CAT GGC TGC CCT ACG GAC AAG CTG GGG CGG TGC AAG      1756
   Val Met Pro Leu His Gly Cys Pro Thr Asp Lys Leu Gly Arg Cys Lys
   430                 435                 440                 445
   CGG GAC GCT TTC GTC GCG GGG CTG AGC TTT GCG CAG GCG GGC GGG AAC      1804
   Arg Asp Ala Phe Val Ala Gly Leu Ser Phe Ala Gln Ala Gly Gly Asn
                   450                 455                 460
   TGG GCG GAT TGT TTC TGATGTTGAG AAGAAAGGTA GATAGATAGG TAGTACATAT      1859
   Trp Ala Asp Cys Phe
               465
   GGATTGCTCG GCTCTGGGTC GTTGCCCACA ATGCATATTA CGCCCGTCAA CTGCCTTGCG    1919
   CCATCCACCT CTCACCCTGG ACGCAACCGA GCGGTCTACC CTGCACACGG CTTCCACCGC    1979
   GACGCGCACG GATAAGGCGC TTTTGTTACG GGGTTGGGGC TGGGGGCAGC CGGAGCCGGA    2039
   GAGAGAGACC AGCGTGAAAA ACGACAGAAC ATAGATATCA ATTCGACGCC AATTCATGCA    2099
   GAGTAGTATA CAGACGAACT GAAACAAACA CATCACTTCC CTCGCTCCTC TCCTGTAGAA    2159
   GACGCTCCCA CCAGCCGCTT CTGGCCCTTA TTCCCGTACG CTAGGTAGAC CAGTCAGCCA    2219
   GACGCATGCC TCACAAGAAC GGGGGCGGGG GACACACTCC GCTCGTACAG CACCCACGAC    2279
   GTGTACAGGA AAACCGGCAG CGCCACAATC GTCGAGAGCC ATCTGCAG                 2327
(2) INFORMATION FOR SEQ ID NO: 2:
   (i) SEQUENCE CHARACTERISTICS:
      (A) LENGTH: 466 amino acids
      (B) TYPE: amino acid
      (D) TOPOLOGY: linear
   (ii) MOLECULE TYPE: protein
   (xi) SEQUENCE DESCRIPTION: SEQ ID NO: 2:
   Met Gly Phe Leu Ala Ile Val Leu Ser Val Ala Leu Leu Phe Arg Ser
    1               5                  10                  15
   Thr Ser Gly Thr Pro Leu Gly Pro Arg Gly Lys His Ser Asp Cys Asn
               20                  25                  30
   Ser Val Asp His Gly Tyr Gln Cys Phe Pro Glu Leu Ser His Lys Trp
           35                  40                  45
   Gly Leu Tyr Ala Pro Tyr Phe Ser Leu Gln Asp Glu Ser Pro Phe Pro
       50                  55                  60
   Leu Asp Val Pro Glu Asp Cys His Ile Thr Phe Val Gln Val Leu Ala
    65                 70                  75                  80
   Arg His Gly Ala Arg Ser Pro Thr His Ser Lys Thr Lys Ala Tyr Ala
                   85                  90                  95
   Ala Thr Ile Ala Ala Ile Gln Lys Ser Ala Thr Ala Phe Pro Gly Lys
               100                 105                 110
   Tyr Ala Phe Leu Gln Ser Tyr Asn Tyr Ser Leu Asp Ser Glu Glu Leu
           115                 120                 125
   Thr Pro Phe Gly Arg Asn Gln Leu Arg Asp Leu Gly Ala Gln Phe Tyr
       130                 135                 140
   Glu Arg Tyr Asn Ala Leu Thr Arg His Ile Asn Pro Phe Val Arg Ala
   145                 150                 155                 160
   Thr Asp Ala Ser Arg Val His Glu Ser Ala Glu Lys Phe Val Glu Gly
                   165                 170                 175
   Phe Gln Thr Ala Arg Gln Asp Asp His His Ala Asn Pro His Gln Pro
               180                 185                 190
   Ser Pro Arg Val Asp Val Ala Ile Pro Glu Gly Ser Ala Tyr Asn Asn
           195                 200                 205
   Thr Leu Glu His Ser Leu Cys Thr Ala Phe Glu Ser Ser Thr Val Gly
       210                 215                 220
   Asp Asp Ala Val Ala Asn Phe Thr Ala Val Phe Ala Pro Ala Ile Ala
   225                 230                 235                 240
   Gln Arg Leu Glu Ala Asp Leu Pro Gly Val Gln Leu Ser Thr Asp Asp
                   245                 250                 255
   Val Val Asn Leu Met Ala Met Cys Pro Phe Glu Thr Val Ser Leu Thr
               260                 265                 270
   Asp Asp Ala His Thr Leu Ser Pro Phe Cys Asp Leu Phe Thr Ala Thr
           275                 280                 285
   Glu Trp Thr Gln Tyr Asn Tyr Leu Leu Ser Leu Asp Lys Tyr Tyr Gly
       290                 295                 300
   Tyr Gly Gly Gly Asn Pro Leu Gly Pro Val Gln Gly Val Gly Trp Ala
   305                 310                 315                 320
   Asn Glu Leu Met Ala Arg Leu Thr Arg Ala Pro Val His Asp His Thr
                   325                 330                 335
   Cys Val Asn Asn Thr Leu Asp Ala Ser Pro Ala Thr Phe Pro Leu Asn
               340                 345                 350
   Ala Thr Leu Tyr Ala Asp Phe Ser His Asp Ser Asn Leu Val Ser Ile
           355                 360                 365
   Phe Trp Ala Leu Gly Leu Tyr Asn Gly Thr Ala Pro Leu Ser Gln Thr
       370                 375                 380
   Ser Val Glu Ser Val Ser Gln Thr Asp Gly Tyr Ala Ala Ala Trp Thr
   385                 390                 395                 400
   Val Pro Phe Ala Ala Arg Ala Tyr Val Glu Met Met Gln Cys Arg Ala
                   405                 410                 415
   Glu Lys Glu Pro Leu Val Arg Val Leu Val Asn Asp Arg Val Met Pro
               420                 425                 430
   Leu His Gly Cys Pro Thr Asp Lys Leu Gly Arg Cys Lys Arg Asp Ala
           435                 440                 445
   Phe Val Ala Gly Leu Ser Phe Ala Gln Ala Gly Gly Asn Trp Ala Asp
       450                 455                 460
   Cys Phe
   465
(2) INFORMATION FOR SEQ ID NO: 3:
   (i) SEQUENCE CHARACTERISTICS:
      (A) LENGTH: 3995 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: join (2208..2263, 2321..3725)
   (xi) SEQUENCE DESCRIPTION: SEQ ID NO: 3:
(2) INFORMATION FOR SEQ ID NO: 4:
   (i) SEQUENCE CHARACTERISTICS:
      (A) LENGTH: 487 amino acids
      (B) TYPE: amino acid
      (D) TOPOLOGY: linear
   (ii) MOLECULE TYPE: protein
   (xi) SEQUENCE DESCRIPTION: SEQ ID NO: 4:
   Met Thr Gly Leu Gly Val Met Val Val Met Val Gly Phe Leu Ala Ile
    1               5                  10                  15
   Ala Ser Leu Gln Ser Glu Ser Arg Pro Cys Asp Thr Pro Asp Leu Gly
               20                  25                  30
   Phe Gln Cys Gly Thr Ala Ile Ser His Phe Trp Gly Gln Tyr Ser Pro
           35                  40                  45
   Tyr Phe Ser Val Pro Ser Glu Leu Asp Ala Ser Ile Pro Asp Asp Cys
       50                  55                  60
   Glu Val Thr Phe Ala Gln Val Leu Ser Arg His Gly Ala Arg Ala Pro
    65                  70                  75                  80
   Thr Leu Lys Arg Ala Ala Ser Tyr Val Asp Leu Ile Asp Arg Ile His
                   85                  90                  95
   His Gly Ala Ile Ser Tyr Gly Pro Gly Tyr Glu Phe Leu Arg Thr Tyr
               100                 105                 110
   Asp Tyr Thr Leu Gly Ala Asp Glu Leu Thr Arg Thr Gly Gln Gln Gln
           115                 120                 125
   Met Val Asn Ser Gly Ile Lys Phe Tyr Arg Arg Tyr Arg Ala Leu Ala
       130                 135                 140
   Arg Lys Ser Ile Pro Phe Val Arg Thr Ala Gly Gln Asp Arg Val Val
   145                 150                 155                 160
   His Ser Ala Glu Asn Phe Thr Gln Gly Phe His Ser Ala Leu Leu Ala
                   165                 170                 175
   Asp Arg Gly Ser Thr Val Arg Pro Thr Leu Pro Tyr Asp Met Val Val
               180                 185                 190
   Ile Pro Glu Thr Ala Gly Ala Asn Asn Thr Leu His Asn Asp Leu Cys
           195                 200                 205
   Thr Ala Phe Glu Glu Gly Pro Tyr Ser Thr Ile Gly Asp Asp Ala Gln
       210                 215                 220
   Asp Thr Tyr Leu Ser Thr Phe Ala Gly Pro Ile Thr Ala Arg Val Asn
   225                 230                 235                 240
   Ala Asn Leu Pro Gly Ala Asn Leu Thr Asp Ala Asp Thr Val Ala Leu
                   245                 250                 255
   Met Asp Leu Cys Pro Phe Glu Thr Val Ala Ser Ser Ser Ser Asp Pro
               260                 265                 270
   Ala Thr Ala Asp Ala Gly Gly Gly Asn Gly Arg Pro Leu Ser Pro Phe
           275                 280                 285
   Cys Arg Leu Phe Ser Glu Ser Glu Trp Arg Ala Tyr Asp Tyr Leu Gln
       290                 295                 300
   Ser Val Gly Lys Trp Tyr Gly Tyr Gly Pro Gly Asn Pro Leu Gly Pro
   305                 310                 315                 320
   Thr Gln Gly Val Gly Phe Val Asn Glu Leu Leu Ala Arg Leu Ala Gly
                   325                 330                 335
   Val Pro Val Arg Asp Gly Thr Ser Thr Asn Arg Thr Leu Asp Gly Asp
               340                 345                 350
   Pro Arg Thr Phe Pro Leu Gly Arg Pro Leu Tyr Ala Asp Phe Ser His
           355                 360                 365
   Asp Asn Asp Met Met Gly Val Leu Gly Ala Leu Gly Ala Tyr Asp Gly
       370                 375                 380
   Val Pro Pro Leu Asp Lys Thr Ala Arg Arg Asp Pro Glu Glu Leu Gly
   385                 390                 395                 400
   Gly Tyr Ala Ala Ser Trp Ala Val Pro Phe Ala Ala Arg Ile Tyr Val
                   405                 410                 415
   Glu Lys Met Arg Cys Ser Gly Gly Gly Gly Gly Gly Gly Gly Gly Glu
               420                 425                 430
   Gly Arg Gln Glu Lys Asp Glu Glu Met Val Arg Val Leu Val Asn Asp
           435                 440                 445
   Arg Val Met Thr Leu Lys Gly Cys Gly Ala Asp Glu Arg Gly Met Cys
       450                 455                 460
   Thr Leu Glu Arg Phe Ile Glu Ser Met Ala Phe Ala Arg Gly Asn Gly
   465                 470                 475                 480
   Lys Trp Asp Leu Cys Phe Ala
                   485
(2) INFORMATION FOR SEQ ID NO: 5:
   (i) SEQUENCE CHARACTERISTICS:
      (A) LENGTH: 102 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 2..100
   (xi) SEQUENCE DESCRIPTION: SEQ ID NO: 5:
   G ACC TTG GCT CGC AAC CAC ACA GAC ACG CTG TCT CCG TTC TGC GCT          46
     Thr Leu Ala Arg Asn His Thr Asp Thr Leu Ser Pro Phe Cys Ala
       1               5                  10                  15
   CTT TCC ACG CAA GAG GAG TGG CAA GCA TAT GAC TAC TAC CAA AGT CTG        94
   Leu Ser Thr Gln Glu Glu Trp Gln Ala Tyr Asp Tyr Tyr Gln Ser Leu
                       20                  25                  30
   GGG AAT AC                                                           102
   Gly Asn
(2) INFORMATION FOR SEQ ID NO: 6:
   (i) SEQUENCE CHARACTERISTICS:
      (A) LENGTH: 33 amino acids
      (B) TYPE: amino acid
      (D) TOPOLOGY: linear
   (ii) MOLECULE TYPE: protein
   (xi) SEQUENCE DESCRIPTION: SEQ ID NO: 6:
   Thr Leu Ala Arg Asn His Thr Asp Thr Leu Ser Pro Phe Cys Ala Leu
     1               5                  10                  15
   Ser Thr Gln Glu Glu Trp Gln Ala Tyr Asp Tyr Tyr Gln Ser Leu Gly
               20                  25                  30
   Asn
(2) INFORMATION FOR SEQ ID NO: 7:
   (i) SEQUENCE CHARACTERISTICS:
      (A) LENGTH: 106 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 2..106
   (xi) SEQUENCE DESCRIPTION: SEQ ID NO: 7:
   T ACG GTA GCG CGC ACC AGC GAC GCA AGT CAG CTG TCA CCG TTC TGT        46
     Thr Val Ala Arg Thr Ser Asp Ala Ser Gln Leu Ser Pro Phe Cys
       1               5                  10                  15
   CAA CTC TTC ACT CAC AAT GAG TGG AAG AAG TAC AAC TAC CTT CAG TCC      94
   Gln Leu Phe Thr His Asn Glu Trp Lys Lys Tyr Asn Tyr Leu Gln Ser
                   20                  25                  30
   TTG GGC AAG TAC                                                     106
   Leu Gly Lys Tyr
               35
(2) INFORMATION FOR SEQ ID NO: 8:
   (i) SEQUENCE CHARACTERISTICS:
      (A) LENGTH: 35 amino acids
      (B) TYPE: amino acid
      (D) TOPOLOGY: linear
   (ii) MOLECULE TYPE: protein
   (xi) SEQUENCE DESCRIPTION: SEQ ID NO: 8:
   Thr Val Ala Arg Thr Ser Asp Ala Ser Gln Leu Ser Pro Phe Cys Gln
     1               5                  10                  15
   Leu Phe Thr His Asn Glu Trp Lys Lys Tyr Asn Tyr Leu Gln Ser Leu
                20                  25                  30
   Gly Lys Tyr
           35
(2) INFORMATION FOR SEQ ID NO: 9:
   (i) SEQUENCE CHARACTERISTICS:
      (A) LENGTH: 109 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 2..109
   (xi) SEQUENCE DESCRIPTION: SEQ ID NO: 9:
   C ACC ATG GCG CGC ACC GCC ACT CGG AAC CGT AGT CTG TCT CCA TTT          46
     Thr Met Ala Arg Thr Ala Thr Arg Asn Arg Ser Leu Ser Pro Phe
       1               5                  10                  15
   TGT GCC ATC TTC ACT GAA AAG GAG TGG CTG CAG TAC GAC TAC CTT CAA        94
   Cys Ala Ile Phe Thr Glu Lys Glu Trp Leu Gln Tyr Asp Tyr Leu Gln
                   20                  25                  30
   TCT CTA TCA AAG TAC                                                   109
   Ser Leu Ser Lys Tyr
               35
(2) INFORMATION FOR SEQ ID NO: 10:
   (i) SEQUENCE CHARACTERISTICS:
      (A) LENGTH: 36 amino acids
      (B) TYPE: amino acid
      (D) TOPOLOGY: linear
   (ii) MOLECULE TYPE: protein
   (xi) SEQUENCE DESCRIPTION: SEQ ID NO: 10:
   Thr Met Ala Arg Thr Ala Thr Arg Asn Arg Ser Leu Ser Pro Phe Cys
     1               5                  10                  15
   Ala Ile Phe Thr Glu Lys Glu Trp Leu Gln Tyr Asp Tyr Leu Gln Ser
               20                  25                  30
   Leu Ser Lys Tyr
           35
(2) INFORMATION FOR SEQ ID NO: 11:
   (i) SEQUENCE CHARACTERISTICS:
      (A) LENGTH: 1912 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION: 1..1396
   (ix) FEATURE:
      (A) NAME/KEY: CDS
      (B) LOCATION: 1..1398
   (xi) SEQUENCE DESCRIPTION: SEQ ID NO: 11:
   ATG GGC GTC TCT GCT GTT CTA CTT CCT TTG TAT CTC CTA GCT GGA GTC      48
   Met Gly Val Ser Ala Val Leu Leu Pro Leu Tyr Leu Leu Ala Gly Val
    1               5                  10                  15
   ACC TCC GGA CTG GCA GTC CCC GCC TCG AGA AAT CAA TCC ACT TGC GAT      96
   Thr Ser Gly Leu Ala Val Pro Ala Ser Arg Asn Gln Ser Thr Cys Asp
               20                  25                  30
   ACG GTC GAT CAA GGG TAT CAA TGC TTC TCC GAG ACT TCG CAT CTT TGG     144
   Thr Val Asp Gln Gly Tyr Gln Cys Phe Ser Glu Thr Ser His Leu Trp
           35                  40                  45
   GGT CAA TAC GCG CCG TTC TTC TCT CTG GCA AAC GAA TCG GTC ATC TCC     192
   Gly Gln Tyr Ala Pro Phe Phe Ser Leu Ala Asn Glu Ser Val Ile Ser
       50                  55                  60
   CCT GAT GTG CCC GCC GGT TGC AGA GTC ACT TTC GCT CAG GTC CTC TCC     240
   Pro Asp Val Pro Ala Gly Cys Arg Val Thr Phe Ala Gln Val Leu Ser
   65                  70                  75                  80
   CGT CAT GGA GCG CGG TAT CCG ACC GAG TCC AAG GGC AAG AAA TAC TCC     288
   Arg His Gly Ala Arg Tyr Pro Thr Glu Ser Lys Gly Lys Lys Tyr Ser
                   85                  90                  95
   GCT CTC ATT GAG GAG ATC CAG CAG AAC GTG ACC ACC TTT GAT GGA AAA     336
   Ala Leu Ile Glu Glu Ile Gln Gln Asn Val Thr Thr Phe Asp Gly Lys
               100                 105                 110
   TAT GCC TTC CTG AAG ACA TAC AAC TAC AGC TTG GGT GCA GAT GAC CTG     384
   Tyr Ala Phe Leu Lys Thr Tyr Asn Tyr Ser Leu Gly Ala Asp Asp Leu
           115                 120                 125
   ACT CCC TTC GGA GAG CAG GAG CTA GTC AAC TCC GGC ATC AAG TTC TAC         432
   Thr Pro Phe Gly Glu Gln Glu Leu Val Asn Ser Gly Ile Lys Phe Tyr
       130                 135                 140
   CAG CGC TAC AAC GCC CTC ACC CGA CAC ATC AAC CCC TTC GTC CGC GCC         480
   Gln Arg Tyr Asn Ala Leu Thr Arg His Ile Asn Pro Phe Val Arg Ala
   145                 150                 155                 160
   ACC GAT GCA TCC CGC GTC CAC GAA TCC GCC GAG AAG TTC GTC GAG GGC         528
   Thr Asp Ala Ser Arg Val His Glu Ser Ala Glu Lys Phe Val Glu Gly
                   165                 170                 175
   TTC CAA ACC GCT CGA CAG GAC GAT CAT CAC GCC AAT CCC CAC CAG CCT         576
   Phe Gln Thr Ala Arg Gln Asp Asp His His Ala Asn Pro His Gln Pro
               180                 185                 190
   TCG CCT CGC GTG GAC GTG GCC ATC CCC GAA GGC AGC GCC TAC AAC AAC         624
   Ser Pro Arg Val Asp Val Ala Ile Pro Glu Gly Ser Ala Tyr Asn Asn
           195                 200                 205
   ACG CTG GAG CAC AGC CTC TGC ACC GCC TTC GAA TCC AGC ACC GTC GGC         672
   Thr Leu Glu His Ser Leu Cys Thr Ala Phe Glu Ser Ser Thr Val Gly
       210                 215                 220
   GAC GAC GCG GTC GCC AAC TTC ACC GCC GTG TTC GCG CCG GCG ATC GCC         720
   Asp Asp Ala Val Ala Asn Phe Thr Ala Val Phe Ala Pro Ala Ile Ala
   225                 230                 235                 240
   CAG CGC CTG GAG GCC GAT CTT CCC GGC GTG CAG CTG TCC ACC GAC GAC         768
   Gln Arg Leu Glu Ala Asp Leu Pro Gly Val Gln Leu Ser Thr Asp Asp
                   245                 250                 255
   GTG GTC AAC CTG ATG GCC ATG TGT CCG TTC GAG ACG GTC AGC CTG ACC         816
   Val Val Asn Leu Met Ala Met Cys Pro Phe Glu Thr Val Ser Leu Thr
               260                 265                 270
   GAC GAC GCG CAC ACG CTG TCG CCG TTC TGC GAC CTC TTC ACG GCC ACT         864
   Asp Asp Ala His Thr Leu Ser Pro Phe Cys Asp Leu Phe Thr Ala Thr
           275                 280                 285
   GAG TGG ACG CAG TAC AAC TAC CTG CTC TCG CTG GAC AAG TAC TAC GGC         912
   Glu Trp Thr Gln Tyr Asn Tyr Leu Leu Ser Leu Asp Lys Tyr Tyr Gly
       290                 295                 300
   TAC GGC GGG GGC AAT CCG CTG GGT CCG GTG CAG GGG GTC GGC TGG GCG         960
   Tyr Gly Gly Gly Asn Pro Leu Gly Pro Val Gln Gly Val Gly Trp Ala
   305                 310                 315                 320
   AAC GAG CTG ATG GCG CGG CTA ACG CGC GCC CCC GTG CAC GAC CAC ACC        1008
   Asn Glu Leu Met Ala Arg Leu Thr Arg Ala Pro Val His Asp His Thr
                   325                 330                 335
   TGC GTC AAC AAC ACC CTC GAC GCG AGT CCG GCC ACC TTC CCG CTG AAC        1056
   Cys Val Asn Asn Thr Leu Asp Ala Ser Pro Ala Thr Phe Pro Leu Asn
               340                 345                 350
   GCC ACC CTC TAC GCC GAC TTC TCC CAC GAC AGC AAC CTG GTG TCG ATC        1104
   Ala Thr Leu Tyr Ala Asp Phe Ser His Asp Ser Asn Leu Val Ser Ile
           355                 360                 365
   TTC TGG GCG CTG GGC CTG TAC AAC GGC ACC GCG CCG CTG TCG CAG ACC      1152
   Phe Trp Ala Leu Gly Leu Tyr Asn Gly Thr Ala Pro Leu Ser Gln Thr
       370                 375                 380
   TCC GTC GAG AGC GTC TCC CAG ACG GAC GGG TAC GCC GCC GCC TGG ACG      1200
   Ser Val Glu Ser Val Ser Gln Thr Asp Gly Tyr Ala Ala Ala Trp Thr
   385                 390                 395                 400
   GTG CCG TTC GCC GCT CGC GCG TAC GTC GAG ATG ATG CAG TGT CGC GCC      1248
   Val Pro Phe Ala Ala Arg Ala Tyr Val Glu Met Met Gln Cys Arg Ala
                   405                 410                 415
   GAG AAG GAG CCG CTG GTG CGC GTG CTG GTC AAC GAC CGG GTC ATG CCG      1296
   Glu Lys Glu Pro Leu Val Arg Val Leu Val Asn Asp Arg Val Met Pro
               420                 425                 430
   CTG CAT GGC TGC CCT ACG GAC AAG CTG GGG CGG TGC AAG CGG GAC GCT      1344
   Leu His Gly Cys Pro Thr Asp Lys Leu Gly Arg Cys Lys Arg Asp Ala
           435                 440                 445
   TTC GTC GCG GGG CTG AGC TTT GCG CAG GCG GGC GGG AAC TGG GCG GAT      1392
   Phe Val Ala Gly Leu Ser Phe Ala Gln Ala Gly Gly Asn Trp Ala Asp
       450                 455                 460
   TGT TTC TGATGTTGAG AAGAAAGGTA GATAGATAGG TAGTACATAT GGATTGCTCG      1448
   Cys Phe
   465
   GCTCTGGGTC GTTGCCCACA ATGCATATTA CGCCCGTCAA CTGCCTTGCG CCATCCACCT    1508
   CTCACCCTGG ACGCAACCGA GCGGTCTACC CTGCACACGG CTTCCACCGC GACGCGCACG    1568
   GATAAGGCGC TTTTGTTACG GGGTTGGGGC TGGGGGCAGC CGGAGCCGGA GAGAGAGACC    1628
   AGCGTGAAAA ACGACAGAAC ATAGATATCA ATTCGACGCC AATTCATGCA GAGTAGTATA    1688
   CAGACGAACT GAAACAAACA CATCACTTCC CTCGCTCCTC TCCTGTAGAA GACGCTCCCA    1748
   CCAGCCGCTT CTGGCCCTTA TTCCCGTACG CTAGGTAGAC CAGTCAGCCA GACGCATGCC    1808
   TCACAAGAAC GGGGGCGGGG GACACACTCC GCTCGTACAG CACCCACGAC GTGTACAGGA    1868
   AAACCGGCAG CGCCACAATC GTCGAGAGCC ATCTGCAGGA ATTC                     1912
(2) INFORMATION FOR SEQ ID NO: 12:
   (i) SEQUENCE CHARACTERISTICS:
      (A) LENGTH: 466 amino acids
      (B) TYPE: amino acid
      (D) TOPOLOGY: linear
   (ii) MOLECULE TYPE: protein
   (xi) SEQUENCE DESCRIPTION: SEQ ID NO: 12:
   Met Gly Val Ser Ala Val Leu Leu Pro Leu Tyr Leu Leu Ala Gly Val
   1               5                   10                  15
   Thr Ser Gly Leu Ala Val Pro Ala Ser Arg Asn Gln Ser Thr Cys Asp
               20                  25                  30
   Thr Val Asp Gln Gly Tyr Gln Cys Phe Ser Glu Thr Ser His Leu Trp
           35                  40                  45
   Gly Gln Tyr Ala Pro Phe Phe Ser Leu Ala Asn Glu Ser Val Ile Ser
       50                  55                  60
   Pro Asp Val Pro Ala Gly Cys Arg Val Thr Phe Ala Gln Val Leu Ser
   65                  70                  75                  80
   Arg His Gly Ala Arg Tyr Pro Thr Glu Ser Lys Gly Lys Lys Tyr Ser
                   85                  90                  95
   Ala Leu Ile Glu Glu Ile Gln Gln Asn Val Thr Thr Phe Asp Gly Lys
               100                 105                 110
   Tyr Ala Phe Leu Lys Thr Tyr Asn Tyr Ser Leu Gly Ala Asp Asp Leu
           115                 120                 125
   Thr Pro Phe Gly Glu Gln Glu Leu Val Asn Ser Gly Ile Lys Phe Tyr
       130                 135                 140
   Gln Arg Tyr Asn Ala Leu Thr Arg His Ile Asn Pro Phe Val Arg Ala
   145                 150                 155                 160
   Thr Asp Ala Ser Arg Val His Glu Ser Ala Glu Lys Phe Val Glu Gly
                   165                 170                 175
   Phe Gln Thr Ala Arg Gln Asp Asp His His Ala Asn Pro His Gln Pro
               180                 185                 190
   Ser Pro Arg Val Asp Val Ala Ile Pro Glu Gly Ser Ala Tyr Asn Asn
           195                 200                 205
   Thr Leu Glu His Ser Leu Cys Thr Ala Phe Glu Ser Ser Thr Val Gly
       210                 215                 220
   Asp Asp Ala Val Ala Asn Phe Thr Ala Val Phe Ala Pro Ala Ile Ala
   225                 230                 235                 240
   Gln Arg Leu Glu Ala Asp Leu Pro Gly Val Gln Leu Ser Thr Asp Asp
                   245                 250                 255
   Val Val Asn Leu Met Ala Met Cys Pro Phe Glu Thr Val Ser Leu Thr
               260                 265                 270
   Asp Asp Ala His Thr Leu Ser Pro Phe Cys Asp Leu Phe Thr Ala Thr
           275                 280                 285
   Glu Trp Thr Gln Tyr Asn Tyr Leu Leu Ser Leu Asp Lys Tyr Tyr Gly
       290                 295                 300
   Tyr Gly Gly Gly Asn Pro Leu Gly Pro Val Gln Gly Val Gly Trp Ala
   305                 310                 315                 320
   Asn Glu Leu Met Ala Arg Leu Thr Arg Ala Pro Val His Asp His Thr
                           325                 330                 335
   Cys Val Asn Asn Thr Leu Asp Ala Ser Pro Ala Thr Phe Pro Leu Asn
               340                 345                 350
   Ala Thr Leu Tyr Ala Asp Phe Ser His Asp Ser Asn Leu Val Ser Ile
           355                 360                 365
   Phe Trp Ala Leu Gly Leu Tyr Asn Gly Thr Ala Pro Leu Ser Gln Thr
       370                 375                 380
   Ser Val Glu Ser Val Ser Gln Thr Asp Gly Tyr Ala Ala Ala Trp Thr
   385                 390                 395                 400
   Val Pro Phe Ala Ala Arg Ala Tyr Val Glu Met Met Gln Cys Arg Ala
                   405                 410                 415
   Glu Lys Glu Pro Leu Val Arg Val Leu Val Asn Asp Arg Val Met Pro
               420                 425                 430
   Leu His Gly Cys Pro Thr Asp Lys Leu Gly Arg Cys Lys Arg Asp Ala
           435                 440                 445
   Phe Val Ala Gly Leu Ser Phe Ala Gln Ala Gly Gly Asn Trp Ala Asp
       450                 455                 460
   Cys Phe
   465
(2) INFORMATION FOR SEQ ID NO: 13:
   (i) SEQUENCE CHARACTERISTICS:
      (A) LENGTH: 112 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 13:
   GACGGTCAGC CTGACCGACG ACGCGCACAC GCTGTCGCCG TTCTGCGACC TCTTCACCGC     60
   CGCCGAGTGG ACGCAGTACA ACTACCTGCT CTCGCTGGAC AAGTACTACG TC            112
(2) INFORMATION FOR SEQ ID NO: 14:
   (i) SEQUENCE CHARACTERISTICS:
      (A) LENGTH: 91 base pairs
      (B) TYPE: nucleic acid
      (C) STRANDEDNESS: double
      (D) TOPOLOGY: linear
   (ii) MOLECULE TYPE: DNA (genomic)
   (xi) SEQUENCE DESCRIPTION: SEQ ID NO: 14:
   CAGTAACCTG GTGTCGATCT TCTGGNCGCT GGGTCTGTAC AACGGCACCA AGCCCCTGTC 60
   GCAGACCACC GTGGAGGATA TCACCCGGAC G 91
(2) INFORMATION FOR SEQ ID NO: 15:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 15:
      ATGGAYATGT GYTCNTTYGA 20
(2) INFORMATION FOR SEQ ID NO: 16:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 16:
      TTRCCRGCRC CRTGNCCRTA 20
(2) INFORMATION FOR SEQ ID NO: 17:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 17:
      TAYGCNGAYT TYTCNCAYGA 20
(2) INFORMATION FOR SEQ ID NO: 18:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 18:
      CGRTCRTTNA CNAGNACNC 19
(2) INFORMATION FOR SEQ ID NO: 19:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 19:
      ATGGAYATGT GYTCNTTYGA 20
(2) INFORMATION FOR SEQ ID NO: 20:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 20:
      TTRCCRGCRC CRTGNCCRTA 20
(2) INFORMATION FOR SEQ ID NO: 21:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 21:
      AGTCCGGAGG TGACTCCAGC TAGGAGATAC 30

## Description

Phytases (*myo*-inositol hexakisphosphate phosphohydrolases; EC 3.1.3.8) are enzymes that hydrolyze phytate (*myo*-inositol hexakisphosphate) to myo-inositol and inorganic phosphate and are known to be valuable feed additives.

A phytase was first described in rice bran in 1907 [Suzuki et al., Bull. Coll. Agr. Tokio Imp. Univ. 7, 495 (1907)] and phytases from Aspergillus species in 1911 [Dox and Golden, J. Biol. Chem. 10, 183-186 (1911)]. Phytases have also been found in wheat bran, plant seeds, animal intestines and in microorganisms [Howsen and Davis, Enzyme Microb. Technol. 5, 377-382 (1983), Lambrechts et al., Biotech. Lett. 14, 61-66 (1992), Shieh and Ware, Appl. Microbiol. 16, 1348-1351 (1968)].

The cloning and expression of the phytase from Aspergillus niger (ficuum) has been described by VanHartingsveldt et al., in Gene, 127, 87-94 (1993) and in European Patent Application, Publication No. 420 358 and from Aspergillus niger var awamori by Piddington et al. in Gene 133, 55-62 (1993).

Since phytases used so far in agriculture have certain disadvantages it is an object of the present invention to provide new phytases or more generally speaking polypeptides with phytase activity against inositol phosphates including phytases ("phytase activity") in large quantities with improved properties. Since it is known that phytases used so far loose activity during the feed pelleting process due to heat treatment, improved heat tolerance would be such a property.

So far phytases have not been reported in thermotolerant fungus with the exception of Aspergillus fumigatus [Dox and Golden et al., J. Biol. Chem. 10, 183-186 (1911)] and Rhizopus oryzae [Howson and Davies, Enzyme Microb. Technol. 5, 377-382 (1993)]. Thermotolerant phosphatases which also show activity regarding phytic acid have been described originating from *Aspergillus terreus* Strain 9A-1 [Yamada et al., Agr. Biol. Chem. 32, 1275-1282 (1968) and Yamamoto et al., Agr. Biol. Chem. Vol 36, No 12, 2097-2103 (1972)] and *Schwanniomyces castellii* [Tempererature optimum 77°C; Segueilha et al., Bioeng. 74, 7-11 (1992)]. The enzyme described in Yamada et al. and Yamamoto et al is a homohexamer with a molecular weight of 214 000 and an enzyme activity for phytic acid with an activity maximum at around ph 4,5. However for commercial use in agriculture such phytases must be available in large quantities. Accordingly it is an object of the present invention to provide DNA sequences coding for heat tolerant phytases. Improved heat tolerance of phytases encoded by such DNA sequences can be determined by assays known in the art, e.g. by the processes used for feed pelleting or assays determing the heat dependence of the enzymatic activity itself as described, e.g. by Yamada et al. (s.a.).

It is furthermore an object of the present invention to screen fungi which show a certain degree of thermotolerance for phytase production. Such screening can be made as described, e.g. in Example 1. In this way heat tolerant fungal strains, listed in Example 1, have been identified for the first time to produce a phytase.

Heat tolerant fungal strains, see e.g. those listed in Example 1, can than be grown as known in the art, e.g. as indicated by their supplier, e.g. the American Tissue Type Culture Collection (ATCC), Deutsche Sammlung von Mikroorganismen und Zellkulturen GmbH (DSM), Agricultural Research Service Culture Collection (NRRL) and the Centralbureau voor Schimmelcultures (CBS) from which such strains are available or as indicated, e.g. in Example 2.

Further improved properties are, e.g. an improved substrate specificity regarding phytic acid [myo-inositol (1,2,3,4,5,6) hexakisphosphate] which is a major storage form of phosphorous in plants and seeds. For the complete release of the six phosphate groups from phytic acid an enzyme is required with sufficient activity against phytic acid and all other inositol phosphate molecules. Using e.g. Aspergillus niger phytase requires for this complete release the addition of the pH 2.5 acid phosphatase. Having only one enzyme with the required activity would be of clear advantage. For example, International Patent Application Publication No. 94/03072 discloses an expression system which allows the expression of a mixture of phytate degrading enzymes in desired ratios. However, it would be even more desirable to have both such activities in a single polypeptide. Therefore it is also an object of the present invention to provide DNA sequences coding for such polypeptides. Phytase and phosphatase activities can be determined by assays known in the state of the art or described, e.g. in Example 9.

Another improved property is, e.g. a so called improved pH-profile. This means, e.g. two phytin degrading activity maxima, e.g. one at around pH 2.5 which could be the pH in the stomach of certain animals and another at around pH 5.5 which could be the pH after the stomach in certain animals. Such pH profile can be determined by assays known in the state of the art or described, e.g. in Example 9. Accordingly it is also an object of the present invention to provide DNA sequences coding for such improved polypeptides.

In general it is an object of the present invention to provide a DNA sequence coding for a polypeptide having phytase activity with an activity maximum at around ph 5,5 and which DNA sequence is derived from a fungus selected from the group consisting of Aspergillus terreus, Aspergillus fumigatus, Aspergillus nidulans, Myceliophthora thermophila, and Talaromyces thermophilus or a DNA sequence coding for a fragment of such a polypeptide which fragment still has phytase activity, or more specifically such a DNA sequence wherein the fungus is selected from the group consisting of Aspergillus fumigatus, Aspergillus nidulans, Aspergillus terreus, Myceliophthora thermophila and Talaromyces thermophilus, or more specifically such a DNA sequence wherein the fungus is selected from the group consisting of Aspergillus terreus, Myceliophthora thermophila, Aspergillus fumigatus, Aspergillus nidulans and Talaromyces thermophilus. DNA sequences coding for a fragment of a polypeptide of the present invention can, e.g. be between 1350 and 900, preferably between 900 and 450 and most preferably between 450 and 150 nucleotides long and can be prepared on the basis of the DNA sequence of the complete polypeptide by recombinant methods or by chemical synthesis with which a man skilled in the art is familiar with.

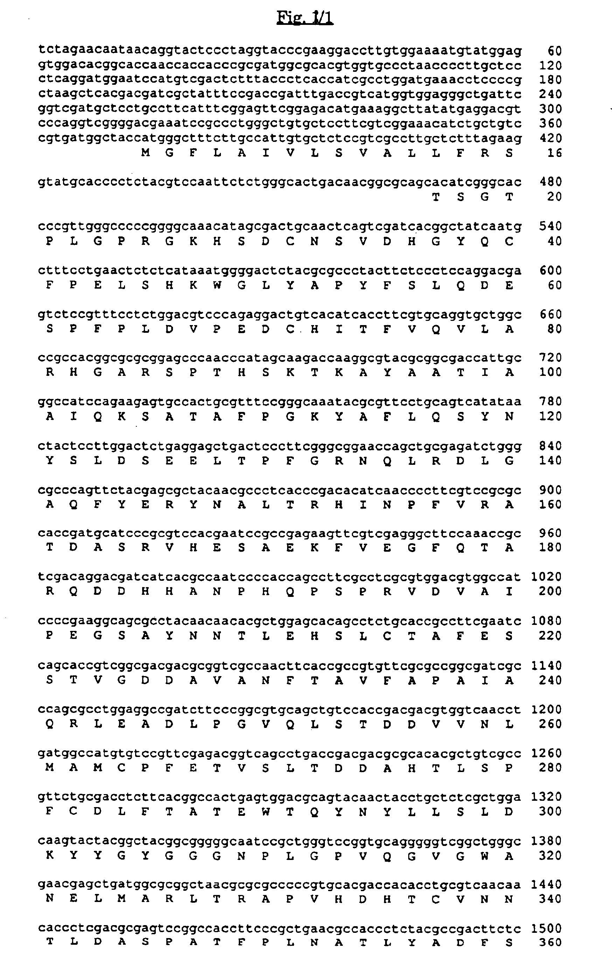

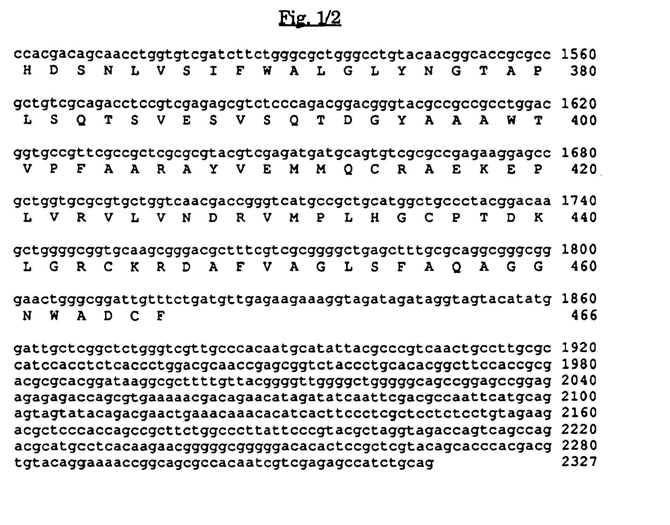

Furthermore it is an object of the present invention to provide a DNA sequence which codes for a polypeptide having phytase activity and which DNA sequence is selected from the following:
(a) the DNA sequence of Figure 1 [SEQ ID NO:1] or its complementary strand;
(b) a DNA sequence which hybridizes under standard conditions with sequences defined under (a) or preferably with the coding region of such sequences or more preferably with a region between positions 491 to 1856 of such DNA sequences or even more preferably with a genomic probe obtained by preferably random priming using DNA of Aspergillus terreus 9A1 as described in Example 12.
(c) a DNA sequence which, because of the degeneracy of the genetic code, does not hybridize with sequences of (a) or (b), but which codes for polypeptides having exactly the same amino acid sequences as the polypeptides encoded by these DNA sequences; and
(d) a DNA sequence which is a fragment of the DNA sequences specified in (a), (b) or (c).

"Standard conditions" for hybridization mean in this context the conditions which are generally used by a man skilled in the art to detect specific hybridization signals and which are described, e.g. by Sambrook et al., "Molecular Cloning" second edition, Cold Spring Harbor Laboratory Press 1989, New York, or preferably so called stringent hybridization and non-stringent washing conditions or more preferably so called stringent hybridization and stringent washing conditions a man skilled in the art is familiar with and which are described, e.g. in Sambrook et al. (s.a.) or even more preferred the stringent hybridization and non-stringent or stringent washing conditions as given in Example 12. "Fragment of the DNA sequences" means in this context a fragment which codes for a polypeptide still having phytase activity as specified above.

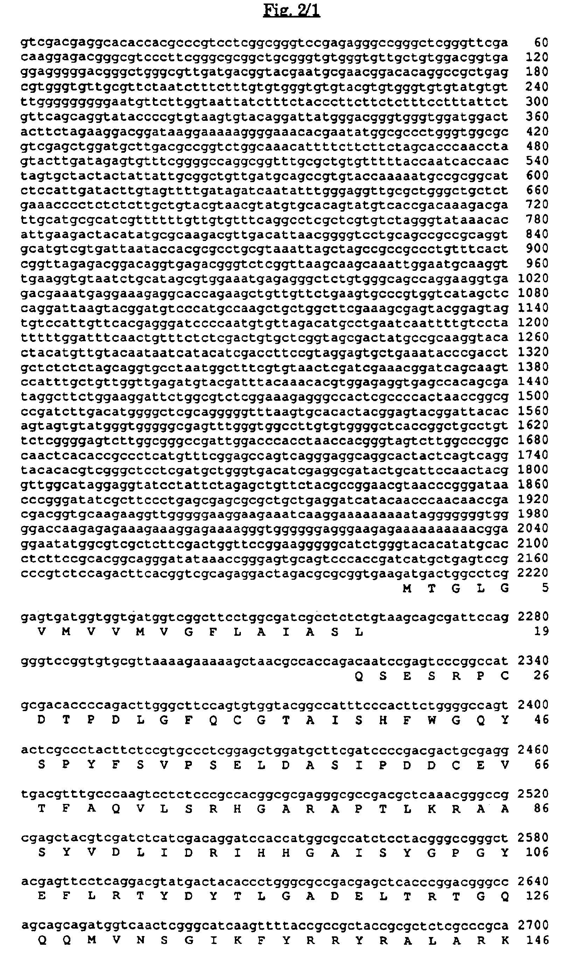

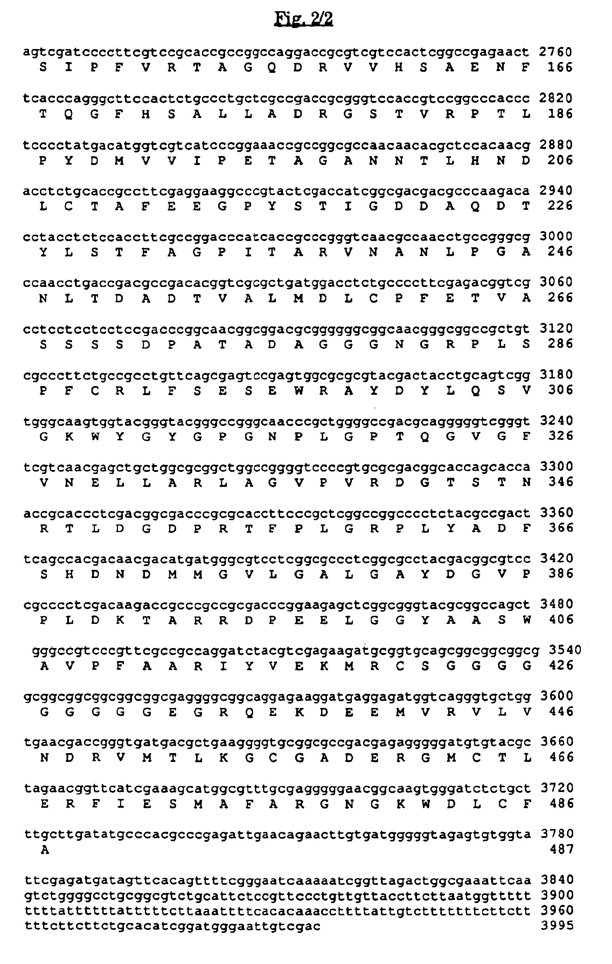

It is also an object of the present invention to provide a DNA sequence which codes for a polypeptide having phytase activity and which DNA sequence is selected from the following:
(a) the DNA sequence of Figure 2 [SEQ ID NO:3] or its complementary strand;
(b) a DNA sequence which hybridizes under standard conditions with sequences defined under (a) or preferably a region which extends to about at least 80 % of the coding region optionally comprising about between 100 to 150 nucleotides of the 5'end of the non-coding region of such DNA sequences or more preferably with a region between positions 2068 to 3478 of such DNA sequences or even more preferably with a genomic probe obtained by preferably random priming using DNA of Myceliophthora thermophila as described in Example 12.
(c) a DNA sequence which, because of the degeneracy of the genetic code, does not hybridize with sequences of (a) or (b), but which codes for polypeptides having exactly the same amino acid sequences as the polypeptides encoded by these DNA sequences; and
(d) a DNA sequence which is a fragment of the DNA sequences specified in (a), (b) or (c).

"Fragments" and "standard conditions" have the meaning as given above.

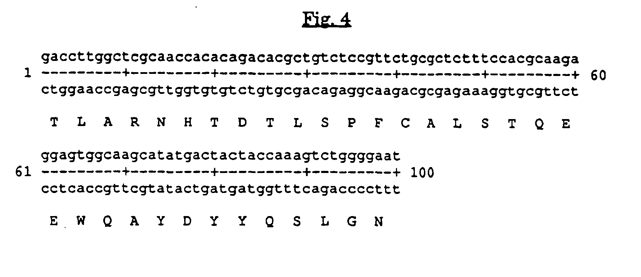

It is also an object of the present invention to provide a DNA sequence which codes for a polypeptide having phytase activity and which DNA sequence is selected from the following:
(a) a DNA sequence comprising one of the DNA sequences of Figures 4 [SEQ ID NO:5], 5 [SEQ ID NO:7], 6 [SEQ ID NO:9] or 10 ["aterr21", SEQ ID NO:13: "aterr58": SEQ ID NO:14] or its complementary strand;
(b) a DNA sequence which hybridizes under standard conditions with sequences defined under (a) or preferably with such sequences comprising the DNA sequence of Figure 4 [SEQ ID NO:5] isolatable from Talaromyces thermophilus, or of Figure 5 [SEQ ID NO:7] isolatable from Aspergillus fumigatus, or of Figure 6 [SEQ ID NO:9] isolatable from Aspergillus nidulans or of one or both of the sequences given in Figure 10 ["aterr21", SEQ ID NO:13: "aterr58": SEQ ID NO:14] isolatable from Aspergillus terreus (CBS 220.95) or more preferably with a region of such DNA sequences spanning at least 80 % of the coding region or most preferably with a genomic probe obtained by random priming using DNA of Talaromyces thermophilus or Aspergillus fumigatus or Aspergillus nidulans or Aspergillus terreus (CBS 220.95) as described in Example 12.
(c) a DNA sequence which, because of the degeneracy of the genetic code, does not hybridize with sequences of (a) or (b) but which codes for polypeptides having exactly the same amino acid sequences as the polypeptides encoded by these DNA sequences; and
(d) a DNA sequence which is a fragment of the DNA sequences specified in (a), (b) or (c).

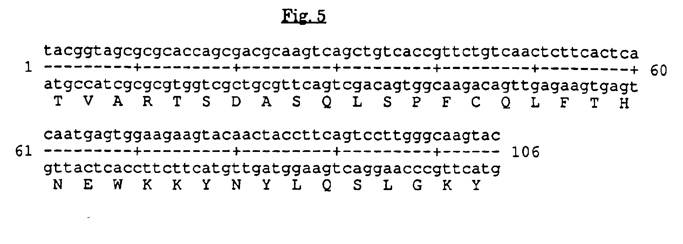

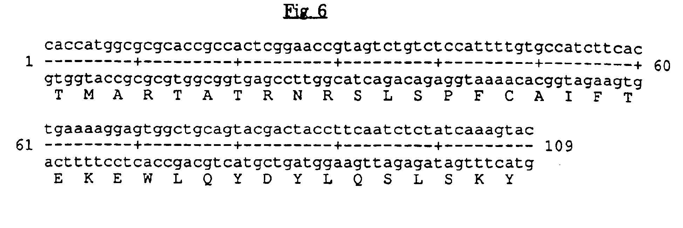

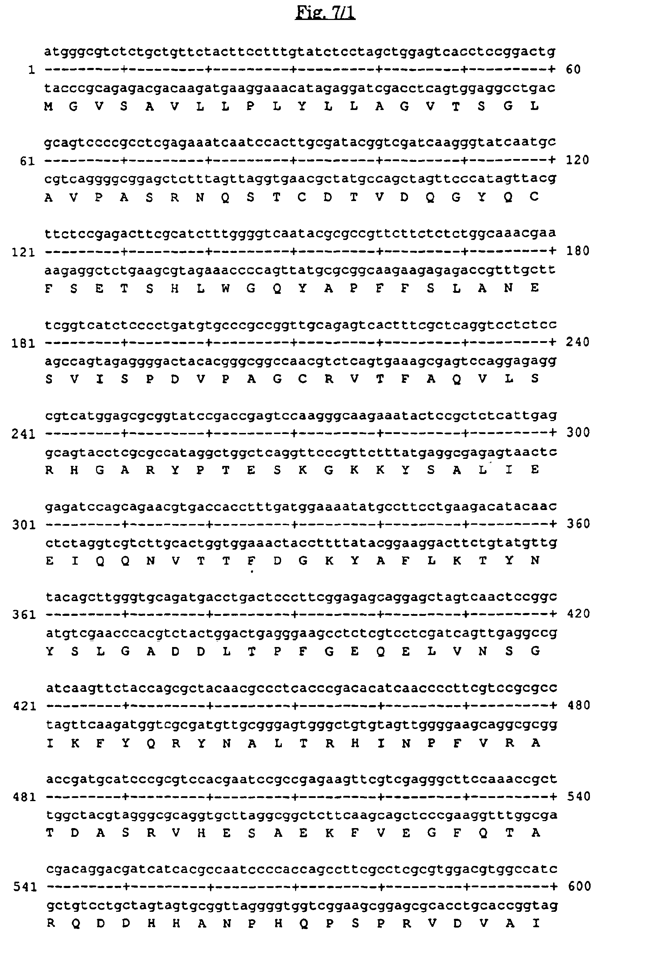

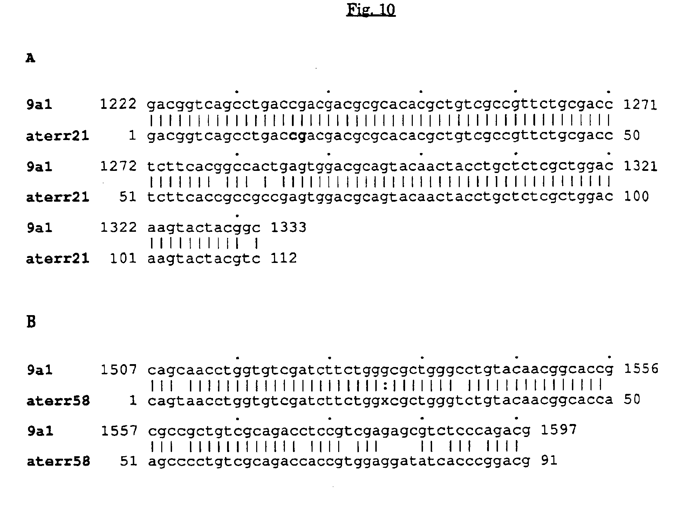

It is furthermore an object of the present invention to provide a DNA sequence which codes for a polypeptide having phytase activity and which DNA sequence is selected from a DNA sequence comprising the DNA sequence of Figure 4 [SEQ ID NO:5] isolatable from Talaromyces thermophilus, of Figure 5 [SEQ ID NO:7] isolatable from Aspergillus fumigatus, of Figure 6 [SEQ ID NO:9] isolatable from Aspergillus nidulans or of Figure 10 ["aterr21": SEQ ID NO:13; "aterr58":SEQ ID NO:14] isolatable from Aspergillus terreus (CBS 220.95) or which DNA sequence is a degenerate variant or equivalent thereof.

"Fragments" and "standard conditions "have the meaning as given above. "Degenerate variant "means in this context a DNA sequence which because of the degeneracy of the genetic code has a different nucleotide sequence as the one referred to but codes for a polypeptide with the same amino acid sequence. "Equivalent" refers in this context to a DNA sequence which codes for polypeptides having phytase activity with an amino acid sequence which differs by deletion, substitution and/or addition of one or more amino acids, preferably up to 50, more preferably up to 20, even more preferably up to 10 or most preferably 5, 4, 3 or 2, from the amino acid sequence of the polypeptide encoded by the DNA sequence to which the equivalent sequence refers to. Amino acid substitutions which do not generally alter the specific activity are known in the state of the art and are described, for example, by H. Neurath and R.L. Hill in "The Proteins" (Academic Press, New York, 1979, see especially Figure 6, page 14). The most commonly occurring exchanges are: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly as well as these in reverse (the three letter abbreviations are used for amino acids and are standard and known in the art).

Such equivalents can be produced by methods known in the state of the art and described, e.g. in Sambrook et al. (s.a.). Whether polypeptides encoded by such equivalent sequences still have a phytase activity can be determined by one of the assays known in the art or, e.g. described in Example 9.

It is also an object of the present invention to provide one of the aforementioned DNA sequences which code for a polypeptide having phytase activity which DNA sequence is derived from a fungus, or more specifically such a fungus selected from one of the above mentioned specific groups of fungi.

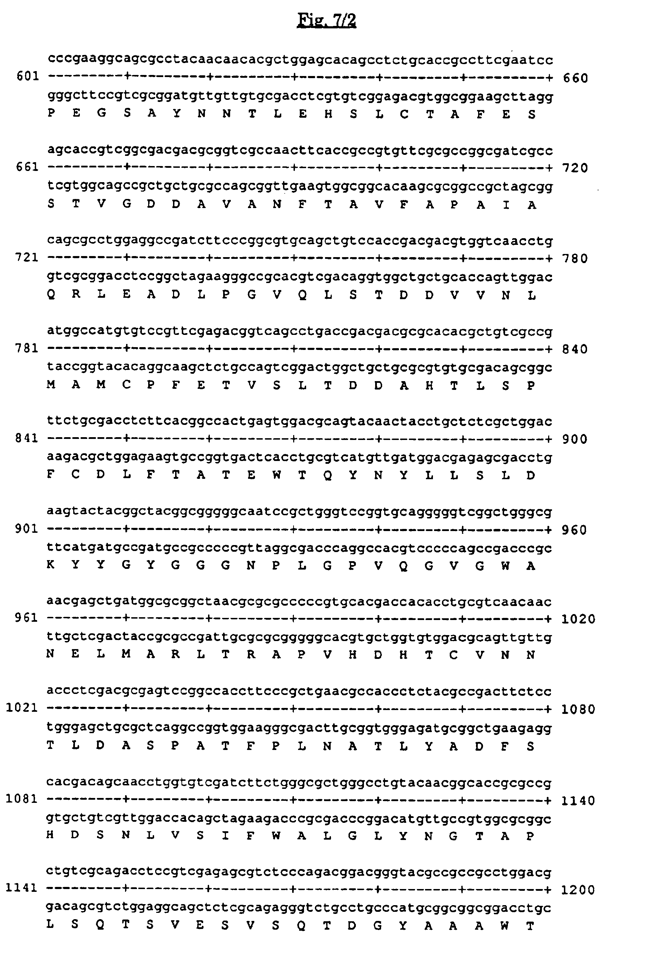

It is furthermore an object of the present invention to provide a DNA sequence coding for a chimeric construct having phytase activity which chimeric construct comprises a fragment of a DNA sequence as specified above or preferably such a DNA sequence wherein the chimeric construct consists at its N-terminal end of a fragment of the Aspergillus niger phytase fused at its C-terminal end to a fragment of the Aspergillus terreus phytase, or more preferably such a DNA sequence with the specific nucleotide sequence as shown in Figure 7 [SEQ ID NO:11] and a degenerate variant or equivalent thereof, wherein "degenerate variant" and "equivalent" have the meanings as given above.

Genomic DNA or cDNA from fungal strains can be prepared as known in the art [see e.g. Yelton et al., Procd. Natl. Acad. Sci. USA, 1470-1474 (1984) or Sambrook et al., s.a., or, e.g. as specifically described in Example 2.

The cloning of the DNA-sequences of the present invention from such genomic DNA can than be effected, e.g. by using the well known polymerase chain reaction (PCR) method. The principles of this method are outlined e.g. by White et al. (1989), whereas improved methods are described e.g. in Innis et al. [PCR Protocols: A guide to Methods and Applications, Academic Press, Inc. (1990)]. PCR is an in vitro method for producing large amounts of a specific DNA of defined length and sequence from a mixture of different DNA-sequences. Thereby, PCR is based on the enzymatic amplification of the specific DNA fragment of interest which is flanked by two oligonucleotide primers which are specific for this sequence and which hybridize to the opposite strand of the target sequence. The primers are oriented with their 3' ends pointing toward each other. Repeated cycles of heat denaturation of the template, annealing of the primers to their complementary sequences and extension of the annealed primers with a DNA polymerase result in the amplification of the segment between the PCR primers. Since the extension product of each primer can serve as a template for the other, each cycle essentially doubles the amount of the DNA fragment produced in the previous cycle. By utilizing the thermostable Taq DNA polymerase, isolated from the thermophilic bacteria Thermus aquaticus, it has been possible to avoid denaturation of the polymerase which necessitated the addition of enzyme after each heat denaturation step. This development has led to the automation of PCR by a variety of simple temperature-cycling devices. In addition, the specificity of the amplification reaction is increased by allowing the use of higher temperatures for primer annealing and extension. The increased specificity improves the overall yield of amplified products by minimizing the competition by non-target fragments for enzyme and primers. In this way the specific sequence of interest is highly amplified and can be easily separated from the non-specific sequences by methods known in the art, e.g. by separation on an agarose gel and cloned by methods known in the art using vectors as described e.g. by Holten and Graham in Nucleic Acid Res. 19,1156 (1991), Kovalic et. al. in Nucleic Acid Res. 19, 4560 (1991), Marchuk et al. in Nucleic Acid Res. 19, 1154 (1991) or Mead et al. in Bio/Technology 9,657-663 (1991).

The oligonucleotide primers used in the PCR procedure can be prepared as known in the art and described e.g. in Sambrook et al. (1989 "Molecular cloning" 2nd edt., Cold Spring Harbor Laboratory Press, Cold Spring Harbor).

The specific primers used in the practice of the present invention have been designed as degenerate primers on the basis of DNA-sequence comparisons of known sequences of the Aspergillus niger phytase, the Aspergillus niger acid phosphatase, the Saccharomyces cerevisiae acid phosphatase and the Schizosaccharomyces pombe acid phosphatase (for sequence information see, e.g. European Bioinformatics Institute (Hinxton Hall, Cambridge, GB). The degeneracy of the primers was reduced by selecting some codons according to a codon usage table of Aspergillus niger prepared on the basis of known sequences from Aspergillus niger. Furthermore it has been found that the amino acid at the C-terminal end of the amino acid sequences used to define the specific probes should be a conserved amino acid in all acid phosphatases including phytases specified above but the rest of the amino acids should be more phytase than phosphatase specific.

Such amplified DNA-sequences can than be used to screen DNA libraries of DNA of, e.g. fungal origin by methods known in the art (Sambrook et al., s.a.) or as specifically described in Examples 5-7.

Once complete DNA-sequences of the present invention have been obtained they can be integrated into vectors by methods known in the art and described e.g. in Sambrook et al. (s.a.) to overexpress the encoded polypeptide in appropriate host systems. However, a man skilled in the art knows that also the DNA-sequences themselves can be used to transform the suitable host systems of the invention to get overexpression of the encoded polypeptide. Appropriate host systems are for example fungi, like Aspergilli, e.g. Aspergillus niger [ATCC 9142] or Aspergillus ficuum [NRRL 3135] or like Trichoderma, e.g. Trichoderma reesei or yeasts, like Saccharomyces, e.g. Saccharomyces cerevisiae or Pichia, like Pichia pastoris, all available from ATCC. Bacteria which can be used are e.g. E. coli, Bacilli as, e.g. Bacillus subtilis or Streptomyces, e.g. Streptomyces lividans (see e.g. Anné and Mallaert in FEMS Microbiol. Letters 114, 121 (1993). E. coli, which could be used are E. coli K12 strains e.g. M15 [described as DZ 291 by Villarejo et al. in J. Bacteriol. 120, 466-474 (1974)], HB 101 [ATCC No. 33694] or E. coli SG13009 [Gottesman et al., J. Bacteriol. 148, 265-273 (1981)].

Vectors which can be used for expression in fungi are known in the art and described e.g. in EP 420 358, or by Cullen et al. [Bio/Technology 5, 369-376 (1987)] or Ward in Molecular Industrial Mycology, Systems and Applications for Filamentous Fungi, Marcel Dekker, New York (1991), Upshall et al. [Bio/Technology 5, 1301-1304 (1987)] Gwynne et al. [Bio/Technology,5, 71-79 (1987)], Punt et al. [J. of Biotechnology 17, 19-34 (1991)] and for yeast by Sreekrishna et al. [J. Basic Microbiol. 28, 265-278 (1988), Biochem. 28, 4117-4125 (1989)], Hitzemann et al. [Nature 293, 717-722 (1981)] or in EP 183 070, EP 183 071, EP 248 227, EP 263 311. Suitable vectors which can be used for expression in E. coli are mentioned, e.g. by Sambrook et al. [s.a.] or by Fiers et al. in Procd. 8th Int. Biotechnology Symposium" [Soc. Franc. de Microbiol., Paris (Durand et al., eds.), pp. 680-697 (1988)] or by Bujard et al. in Methods in Enzymology, eds. Wu and Grossmann, Academic Press, Inc. Vol. 155, 416-433 (1987) and Stüber et al. in Immunological Methods, eds. Lefkovits and Pernis, Academic Press, Inc., Vol. IV, 121-152 (1990). Vectors which could be used for expression in Bacilli are known in the art and described, e.g. in EP 405 370, Procd. Nat. Acad. Sci. USA 81, 439 (1984) by Yansura and Henner, Meth. Enzym. 185, 199-228 (1990) or EP 207 459.

Either such vectors already carry regulatory elements, e.g. promotors or the DNA-sequences of the present invention can be engineered to contain such elements. Suitable promotor-elements which can be used are known in the art and are, e.g. for Trichoderma reesei the cbhl- [Haarki et al., Biotechnology 7, 596-600 (1989)] or the pki1-promotor [Schindler et al., Gene 130, 271-275 (1993)], for Aspergillus oryzae the amy-promotor [Christensen et al., Abstr. 19th Lunteren Lectures on Molecular Genetics F23 (1987), Christensen et al., Biotechnology 6, 1419-1422 (1988), Tada et al., Mol. Gen. Genet. 229, 301 (1991)], for Aspergillus niger the glaA- [Cullen et al., Bio/Technology 5, 369-376 (1987), Gwynne et al., Bio/Technlogy 5, 713-719 (1987), Ward in Molecular Industrial Mycology, Systems and Applications for Filamentous Fungi, Marcel Dekker, New York, 83-106 (1991)], alcA- [Gwynne et al., Bio/Technology 5, 71-719 (1987)], suc1- [Boddy et al. Current Genetics 24, 60-66 (1993)], aphA- [MacRae et al., Gene 71, 339-348 (1988), MacRae et al., Gene 132, 193-198 (1993)], tpiA- [McKnight et al., Cell 46, 143-147 (1986), Upshall et al., Bio/Technology, 5, 1301-1304 (1987)], gpdA- [Punt et al., Gene 69, 49-57 (1988), Punt et al., J. of Biotechnology 17, 19-37 (1991)] and the pkiA-promotor [de Graaff et al., Curr. Genet. 22, 21-27 (1992)]. Suitable promotor-elements which could be used for expression in yeast are known in the art and are, e.g. the pho5-promotor [Vogel et al., Molecular and Cellular Biology, 2050-2057 (1989); Rudolf and Hinnen, Proc. Natl. Acad. Sci. 84,1340-1344 (1987)] or the gap-promotor for expression in Saccharamyces cerevisiae und for Pichia pastoris, e.g. the aoxl-promotor [Koutz et al. Yeast 5, 167-177 (1989); Sreekrishna et al., J. Basic Microbiol. 28, 265-278 (1988)].

Accordingly vectors comprising DNA sequences of the present invention, preferably for the expression of said DNA sequences in bacteria or a fungal or a yeast host and such transformed bacteria or fungal or yeast hosts are also an object of the present invention.

Once such DNA-sequences have been expressed in an appropriate host cell in a suitable medium the encoded phytase can be isolated either from the medium in the case the phytase is secreted into the medium or from the host organism in case such phytase is present intracellularly by methods known in the art of protein purification or described, e.g. in EP 420 358. Accordingly a process for the preparation of a polypeptide of the present invention characterized in that transformed bacteria or a host cell as described above is cultured under suitable culture conditions and the polypeptide is recovered therefrom and a polypeptide when produced by such a process or a polypeptide encoded by a DNA sequence of the present invention are also an object of the present invention.

Once obtained the polypeptides of the present invention can be characterized regarding their activity by assays known in the state of the art or as described, e.g. by Engelen et al. [J. AOAC Intern. 77, 760-764 (1994)] or in Example 9. Regarding their properties which make the polypeptides of the present invention useful in agriculture any assay known in the art and described e.g. by Simons et al. [British Journal of Nutrition 64, 525-540 (1990)], Schöner et al. [J. Anim. Physiol. a. Anim. Nutr. 66, 248-255 (1991)], Vogt [Arch. Geflügelk. 56, 93-98 (1992)], Jongbloed et al. [J. Anim. Sci., 70, 1159-1168 (1992)], Perney et al. [Poultry Science 72, 2106-2114 (1993)], Farrell et al., [J. Anim. Physiol. a. Anim. Nutr. 69, 278-283 (1993), Broz et al., [British Poultry Science 35, 273-280 (1994)] and Düngelhoef et al. [Animal Feed Science and Technology 49, 1-10 (1994)] can be used. Regarding their thermotolerance any assay known in the state of the art and described, e.g. by Yamada et al. (s.a.), and regarding their pH and substrate specificity profiles any assays known in the state of the art and described, e.g. in Example 9 or by Yamada et al., s.a., can be used.

In general the polypeptides of the present invention can be used without being limited to a specific field of application for the conversion of phytate to inositol and inorganic phosphate.

Furthermore the polypeptides of the present invention can be used in a process for the preparation of compound food or feeds wherein the components of such a composition are mixed with one or more polypeptides of the present invention. Accordingly compound food or feeds comprising one or more polypeptides of the present invention are also an object of the present invention. A man skilled in the art is familiar with their process of prepration. Such compound foods or feeds can further comprise additives or components generally used for such purpose and known in the state of the art.

It is furthermore an object of the present invention to provide a process for the reduction of levels of phytate in animal manure characterized in that an animal is fed such a feed composition in an amount effective in converting phytate contained in the feedstuff to inositol and inorganic phosphate.

### Examples

### Specific media and solutions used

### Complete medium (Clutterbuck)

| | |
|---|---|
| Glucose | 10 g/l |
| -CN solution | 10 ml/l |
| Sodium nitrate | 6 g/l |
| Bacto peptone (Difco Lab., Detroit, MI, USA) | 2 g/l |
| Yeast Extract (Difco) | 1 g/l |
| Casamino acids (Difco) | 1.5 g/l |
| Modified trace element solution | 1 ml/l |
| Vitamin solution | 1 ml/l |

### M3 Medium

| | |
|---|---|
| Glucose | 10 g/l |
| -CN Solution | 10 ml/l |
| Modified trace element solution | 1 ml/l |
| Ammonium nitrate | 2 g/l |

### M3 Medium - Phosphate

### M3 medium except that -CN is replaced with -CNP

### M3 Medium - Phosphate + Phytate

### M3 Medium - Phosphate with the addition of 5g/l of Na₁₂ Phytate (Sigma #P-3168; Sigma, St. Louis, MO, USA)

### Modified trace element solution

| | |
|---|---|
| CuSO4 | 0.04% |
| FeSO4·7H₂O | 0.08% |
| Na₂MoO₄·2H₂O | 0.08% |
| ZnSO₄·7H₂O | 0.8% |
| B₄Na₂O₇·10H₂O | 0.004% |
| MnS₄·H₂O | 0.08% |

### Vitamin Solution

| | |
|---|---|
| Riboflavin | 0.1% |
| Nicotinamide | 0.1% |
| p-amino benzoic acid | 0.01% |
| Pyridoxine/HCl | 0.05% |
| Aneurine/HCl | 0.05% |
| Biotin | 0.001% |

### -CN Solution

| | |
|---|---|
| KH₂PO₄ | 140 g/l |
| K₂PO₄·3H₂O | 90 g/l |
| KCl | 10 g/l |
| NIgSO₄·7H₂O | 10 g/l |

### -CNP Solution

| | |
|---|---|
| HEPES | 47.6g/200 mls |
| KCl | 2 g/200 mls |
| MgSO₄·7H₂O | 2 g/200 mls |

### Example 1

### Screening fungi for phytase activity

Fungi were screened on a three plate system, using the following three media:
"M3" (a defined medium containing phosphate),
"M3-P" (M3 medium lacking phosphate) and
"M3-P+Phytate" (M3 medium lacking phosphate but containing phytate as a sole phosphorus source).

Plates were made with agarose to decrease the background level of phosphate.

Fungi were grown on the medium and at the temperature recommended by the supplier. Either spores or mycelium were transfered to the test plates and incubated at the recommended temperature until growth was observed.

The following thermotolerant strains were found to exhibit such growth:
*Myceliophthora thermophila* [ATCC 48 102]
*Talaromyces thermophilus* [ATCC 20 186]
*Aspergillus fumigatus* [ATCC 34 625]

### Example 2

### Growth of fungi and preparation of genomic DNA

Strains of Myceliophthora thermophila, Talaromyces thermophilus, Aspergillus fumigatus, Aspergillus nidulans, Aspergillus terreus 9A-1, and Aspergillus terreus CBS 220.95 were grown in Potato Dextrose Broth (Difco Lab., Detroit, MI, USA) or complete medium (Clutterbuck). Aspergillus terreus 9A-1 and Aspergillus nidulans have been deposited under the Budapest Treaty for patent purposes at the DSM in Braunschweig, BRD at March 17, 1994 under accession number DSM 9076 and at February 17, 1995 under accession number DSM 9743, respectively.

### Genomic DNA was prepared as follows:

Medium was innoculated at a high density with spores and grown O/N with shaking. This produced a thick culture of small fungal pellets. The mycelium was recovered by filtration blotted dry and weighed. Up to 2.0g was used per preparation. The mycelium was ground to a fine powder in liquid nitrogen and immediately added to 10 mls of extraction buffer (200 mM Tris/HCl, 250 mM NaCl, 25 mM EDTA, 0.5% SDS, pH 8.5) and mixed well. Phenol (7 mls) was added to the slurry and mixed and then chloroform (3 mls) was also added and mixed well. The mixture was centrifuged (20,000 g) and the aqueous phase recovered. RNase A was added to a final concentration of 250 µg/ml and incubated at 37°C for 15 minutes. The mixture was then extracted with 1 volume of chloroform and centrifuged (10,000 g, 10 minutes). The aqueous phase was recovered and the DNA precipitated with 0.54 volumes of RT isopropanol for 1 hour at RT. The DNA was recovered by spooling and resuspended in water.

The resultant DNA was further purified as follows:

A portion of the DNA was digested with proteinase K for 2 hrs at 37°C and then extracted repeatedly (twice to three times) with an equal volume of phenol/chloroform and then ethanol precipitated prior to resuspension in water to a concentration of approximately 1 µg/µl.

### Example 3

### Degenerate PCR

PCR was performed essentially according to the protocol of Perkin Elmer Cetus [(PEC); Norwalk, CT, USA]. The following two primers were used (bases indicated in brackets are either/or):
Phyt 8: 5' ATG GA(CT) ATG TG(CT) TCN TT(CT) GA 3' [SEQ ID NO:19]
   Degeneracy = 32
   Tm High = 60°C/ Tm Low 52°C
Phyt 9: 5' TT(AG) CC(AG) GC(AG) CC(GA) TGN CC(GA) TA 3' [SEQ ID NO:20]
   Tm High = 70°C/ Tm Low 58°C

A typical reaction was performed as follows:

| | |
|---|---|
| H₂O | 24.5 µl |
| 10 X PEC GeneAmp Buffer | 5 µl |
| GeneAmp dNTP's (10 mM) | 8 µl |
| Primer 1 (Phyt 8, 100 µM) | 5 µl |
| Primer 2 (Phyt 9, 100 µM) | 5 µl |
| DNA (~1 µg/µl) | 1 µl |
| Taq Polymerase (PEC) | 0.5 µl |
| | 50 µl |

All components with the exception of the Taq polymerase were incubated at 95 °C for 10 minutes and then 50°C for 10 minutes and then the reaction placed on ice. The Taq polymerase (Amplitaq, Hoffmann-La Roche, Basel, CH) was then added and 35 cycles of PCR performed in a Triothermoblock (Biometra, Göttingen, DE) according to the following cycle profile:
95 °C/ 60"
50 °C/ 90"
72 °C/ 120"

An aliquot of the reaction was analysed on 1.5% agarose gel.

### Example 4

### Subcloning and sequencing of PCR fragments

PCR products of the expected size (approximately 146 bp predicted from the Aspergillus niger DNA-sequence) were excised from low melting point agarose and purified from a NACS - PREPAC - column (BRL Life Technologies Inc., Gaithersburg, MD, USA) essentially according to the manufacturer's protocol. The fragment was polyadenylated in 50 µl 100 mM Sodiumcacodylate pH6.6, 12.5 mM Tris/HCl pH 7.0, 0.1 mM Dithiothreitol, 125 µg/ml bovine serum albumin, 1 mM CoCl₂, 20 µMdATP, 10 units terminal deoxytransferase (Boehringer Mannheim, Mannheim, DE) for 5 minutes at 37°C and cloned into the p123T vector [Mitchell et al., PCR Meth. App. 2, 81-82 (1992)].

Alternatively, PCR fragments were purified and cloned using the "Sure Clone" ligation kit (Pharmacia) following the manufacturers instructions.

Sequencing was performed on dsDNA purified on a Quiagen-column (Diagen GmbH, Hilden, DE) using the dideoxy method and the Pharmacia T7 kit (Pharmacia, LKB Biotechnology AB, Uppsala, SE) according to the protocol supplied by the manufacturer.

### Example 5

### Construction and Screening of Lambda Fix II libraries

The fragments from *Aspergillus terreus* Strain 9A-1 and *Myceliophthora thermophila* were used to probe Bam HI and BglII southerns to determine the suitable restriction enzyme to use to construct genomic libraries in the Lambda Fix II vector (Strategene, La Jolla, CA, USA). Lambda Fix II can only accept inserts from 9-23 kb. Southerns were performed according to the following protocol. Genomic DNA (10 µg) was digested in a final volume of 200 µl. The reaction without enzyme was prepared and incubated on ice for 2 hours. The enzyme (50 units) was added and the reaction incubated at the appropriate temperature for 3 hours. The reaction was then extracted with an equal volume of phenol/chloroform and ethanol precipitated. The resuspended DNA in loading buffer was heated to 65 °C for 15 minutes prior to separation on a 0.7% agarose gel (O/N 30 V). Prior to transfer the gel was washed twice in 0.2 M HCl/ 10'/room temperature (RT) and then twice in 1M NaCl/0.4M NaOH for 15' at RT. The DNA was transfered in 0.4M NaOH in a capillary transfer for 4 hours to Nytran 13N nylon membrane (Schleicher and Schuell AG, Feldbach, Zurich, CH). Following transfer the membrane was exposed to UV. [Auto cross-link, UV Stratalinker 2400, Stratagene (La Jolla, CA, USA)].

The membrane was prehybridized in hybridization buffer [50 % formamide, 1% sodium dodecylsulfate (SDS), 10% dextransulfate, 4 x SSPE (180 mM NaCl, 10 mM NaH₂ PO₄, 1 mM EDTA, ph 7.4)] for 4 hours at 42 °C and following addition of the denatured probe O/N at 42 °C. The blot was washed:
1 x SSPE/0.5 % SDS / RT/ 30 minutes
0.1 x SSPE/0.1 % SDS RT/ 30 minutes
0.1 x SSPE/0.1 % SDS / 65 °C/ 30 minutes

Results indicate that *Aspergillus terreus* Strain 9A-1 genomic DNA digested with BamHI and *Myceliophthora thermophila* genomic DNA digested with BglII produce fragments suitable for cloning into the lambda Fix II vector.

The construction of genomic libraries of *Aspergillus terreus* Strain 9A-1 and *Myceliophthora thermophila* in Lambda Fix II was performed according to the manufacturer's protocols (Stratagene).

The lambda libraries were plated out on 10 137 mm plates for each library. The plaques were lifted to Nytran 13N round filters and treated for 1 minute in 0.5 M NaOH/1.5 M NaCl followed by 5 minutes in 0.5 M Tris-HCl pH 8.0/1.5 M NaCl. The filters were then treated in 2 X SSC for 5 minutes and air dried. They were then fixed with UV (1 minute, UV Stratalinker 2400, Stratagene). The filters were hybridized and washed as above. Putative positive plaques were cored and the phage soaked out in SM buffer (180 mM NaCl, 8 mM MgSO₄·7H₂O, 20mMTris/HCl pH 7.5, 0.01% gelatin). This stock was diluted and plated out on 137 mm plates. Duplicate filters were lifted and treated as above. A clear single positive plaque from each plate was picked and diluted in SM buffer. Three positive plaques were picked. Two from *Aspergillus terreus* Strain 9A-1 (9A1λ17 and 9A1λ22) and one from *Myceliophthora thermophila* (MTλ27).

### Example 6

### Preparation of Lambda DNA and confirmation of the clones

Lambda DNA was prepared from the positive plaques. This was done using the "Magic Lambda Prep" system (Promega Corp., Madison, WI, USA) and was according to the manufactures specifications. To confirm the identity of the clones, the lambda DNA was digested with PstI and SalI and the resultant blot probed with the PCR products. In all cases this confirmed the clones as containing sequences complementary to the probe.

### Example 7

### Subcloning and sequencing of phytase genes

DNA from 9A1λ17 was digested with PstI and the resultant mixture of fragments ligated into pBluescript II SK+ (Stratagene) cut with PstI and treated with shrimp alkaline phosphatase (United States Biochemical Corp., Cleaveland, OH, USA). The ligation was O/N at 16 °C. The ligation mixture was transformed into XL-1 Blue Supercompetent cells (Stratagene) and plated on LB Plates containing 0.5 mM isopropyl-β-D-thiogalactopyranoside (IPTG), 40 µg/ml 5-bromo-4-chloro-3-indoyl-β-D-galactopyranoside (Xgal), 50 µg/ml ampicillin.

DNA from 9Aλ17 was digested with Bgl II and Xba I and the resultant mixture ligated into pBluescript II SK+ digested with BamHI/Xba I. Ligation, transformation and screening were performed as described above.

DNA from MTλ27 was digested with SalI and the resultant mixture of fragments ligated into pBluescript II SK+ cut with SalI and treated with shrimp alkaline phosphatase. The ligation was O/N at 16 °C. The ligation mixture was transformed into XL-1 Blue Supercompetent cells and plated on LB Plates containing Xgal/TPTG and ampicillin.

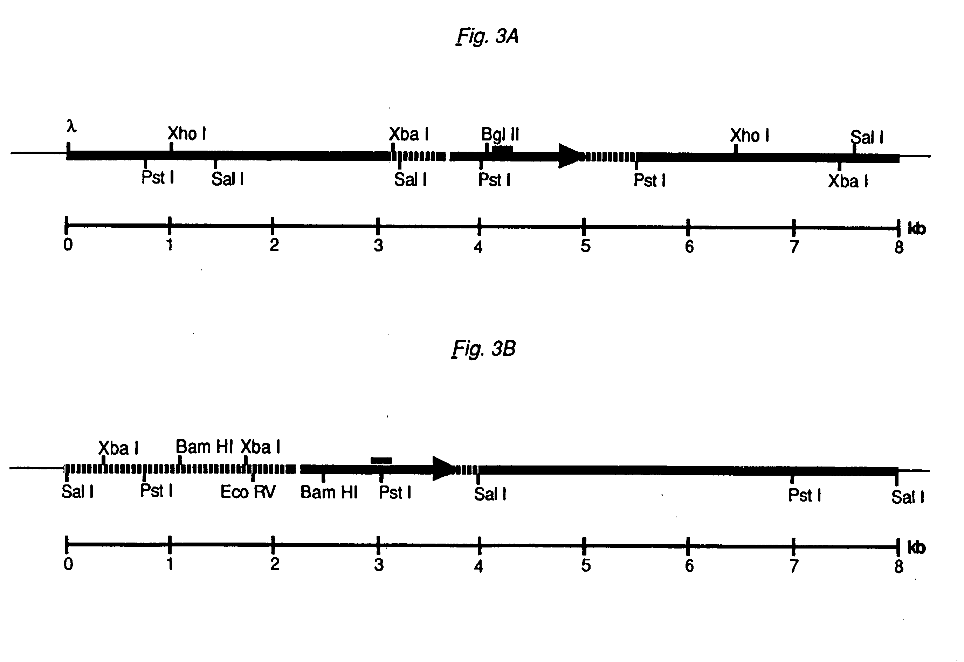

Colonies from the above transformations were picked and "gridded" approximately 75 to a single plate. Following O/N incubation at 37 °C the colonies were lifted to a nylon filter ("Hybond-N", Amersham Corp., Arlington Heights, IL, USA) and the filters treated with 0.5M NaOH for 3 minutes, 1M Tris/HCl pH7.5 twice for 1 minute, then 0.5M Tris/HCl pH7.5/1.5 M NaCl for 5 minutes. The filters were air dried and then fixed with UV (2 minutes, UV Stratalinker 2400, Stratagene). The filters were hybridized with the PCR products of Example 5. Positive colonies were selected and DNA prepared. The subclones were sequenced as previously described in Example 4. Sequences determined are shown in Figure 1 (Fig. 1) for the phytase from Aspergillus terreus strain 9A1 and its encoding DNA sequence, Figure 2 for the phytase from Myceliophthora thermophila and its encoding DNA-sequence, Figure 3A shows a restriction map for the DNA of Aspergillus terreus (wherein the arrow indicates the coding region, and the strips the regions sequenced in addition to the coding region) and 3B for M. thermophila, and Figure 4 for part of the phytase from Talaromyces thermophilus and its encoding DNA sequence, Figure 5 for part of the phytase from Aspergillus fumigatus and its encoding DNA-sequence and Figure 6 for part of the phytase from Aspergillus nidulans and its encoding DNA-sequence. The sequences for the parts of the phytases and their encoding DNA-sequences from Talaromyces thermophilus, Aspergillus fumigatus and Aspergillus nidulans were obtained in the same way as described for those of Aspergillus terreus strain 9A1 and Myceliophthora thermophila in Examples 2-7. Bases are given for both strands in small letters by the typically used one letter code abbreviations. Derived amino acid sequences of the phytase are given in capital letters by the typically used one letter code below the corresponding DNA-sequence.

### Example 8

### Construction of a chimeric construct between A. niger and A. terreus phytase DNA-sequences

All constructions were made using standard molecular biological procedures as described by Sambrook et al., (1989) (Molecular cloning, A laboratory Manual, Cold Spring Harbor Laboratory Press, NY).

The first 146 amino acids (aa) of the *Aspergillus niger* phytase, as described in EP 420 358, were fused to the 320 C-terminal aa of the *Aspergillus terreus* 9A1 gene. A NcoI site was introduced at the ATG start codon when the A. *niger* phytase gene was cloned by PCR. The intron found in the A. niger phytase was removed by site directed mutagenesis (Bio-Rad kit, Cat Nr 170-3581; Bio-Rad, Richmond, CA, USA) using the following primer (werein the vertical dash indictes that the sequence to its left hybridizes to the 3'end of the first exon and the sequence to its right hybridizes to the 5'end of the second exon):
5'-AGTCCGGAGGTGACT | CCAGCTAGGAGATAC-3' [SEQ ID NO:21].

To construct the chimeric construct of phytases from A. niger and A. terreus an Eco 47III site was introduced into the A. niger coding sequence to aid cloning. PCR with a mutagenic primer (5' CGA TTC GTA gCG CTG GTA G 3') in conjunction with the T3 primer was used to produce a DNA fragment that was cleaved with Bam HI and Eco 47III. The Bam HI/Eco 47III fragment was inserted into Bam HI/Eco 47III cut p9A1Pst (Example 7). Figure 7 shows the amino acid sequence of the fusion construct and its encoding DNA-sequence.

### Example 9

### Expression of phytases

### Construction of expression vectors

For expression of the fusion construct in *A. niger* an expression cassette was chosen where the fusion gene was under control of the inducible *A. niger* glucoamylase (*glaA*) promoter.

For the complete *A. terreus* 9A1 gene, expression cassettes with the constitutive *A. nidulans* glyceraldehyde-3-phosphate dehydrogenase (*gpdA*) promoter were made.

All genes used for expression in *A. niger* carried their own signal sequence for secretion.

### Construction of vector pFPAN1

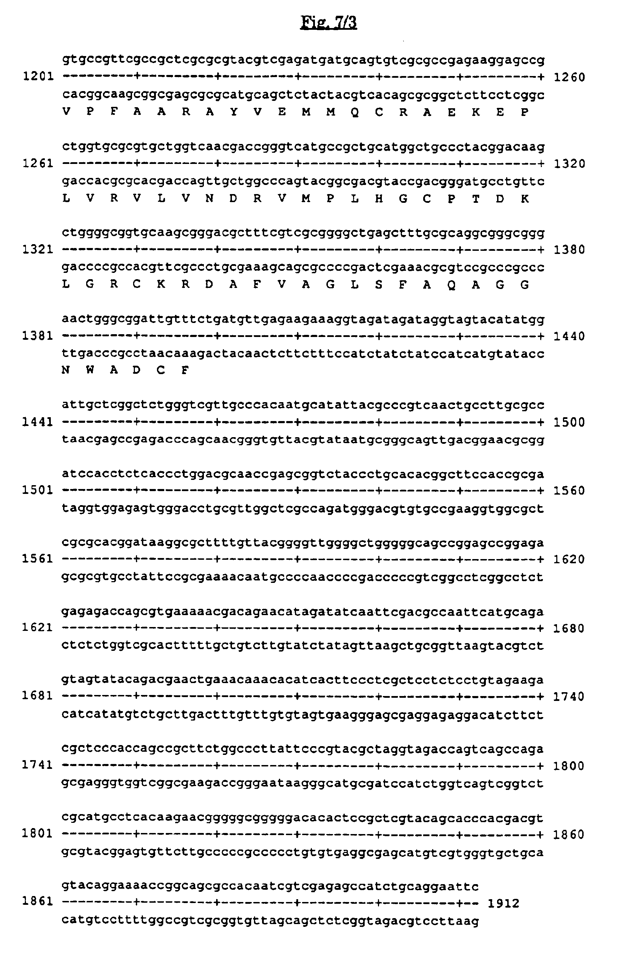

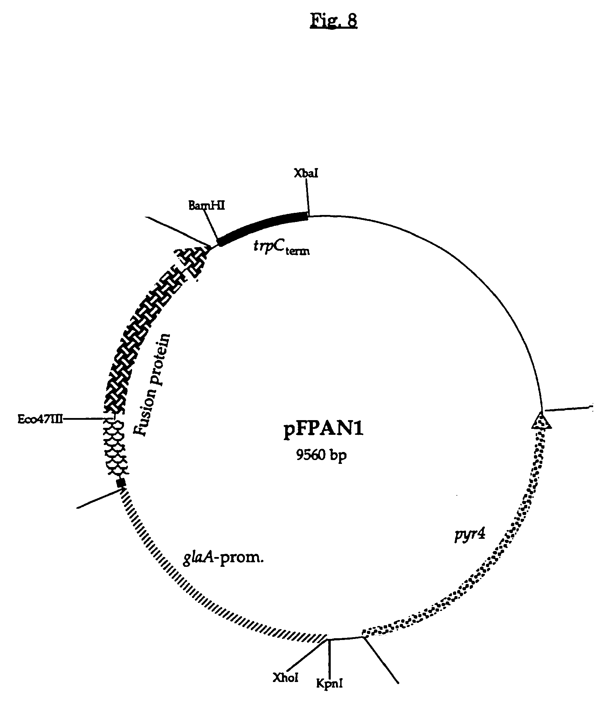

The A. *niger* glucoamylase (*glaA*) promoter was isolated as a 1960 bp XhoI/ClaI fragment from plasmid pDH33 (Smith et al. (1990), Gene 88: 259-262] and cloned into pBluescriptSK⁺-vector (pBS) [Stratagene, La Jolla, CA, USA] containing the 710 bp BamHI/XbaI fragment of the *A. nidulans trpC* terminator. The plasmid with the cassette was named pGLAC. The fusion gene, as described in Example 8, was put under control of the *A. niger glaA* promoter by ligating the blunt ended NcoI/EcoRI fragment to the blunt ended ClaI site and the EcoRV site of plasmid pGLAC. The correct orientation was verified by restriction enzyme digests. The entire cassette was transferred as a KpnI/XbaI fragment to pUC19 (New England Biolabs, GmbH, Schwalbach, BRD), that carried the *Neurospora crassa pyr4* gene (pUC19-pyr4), a selection marker in uridine auxotrophic *Aspergilli,* resulting in vector pFPAN1 (see Figure 8 with restriction sites and coding regions as indicated; crossed out restriction sites indicate sites with blunt end ligation).

### Construction of vector pPAT1

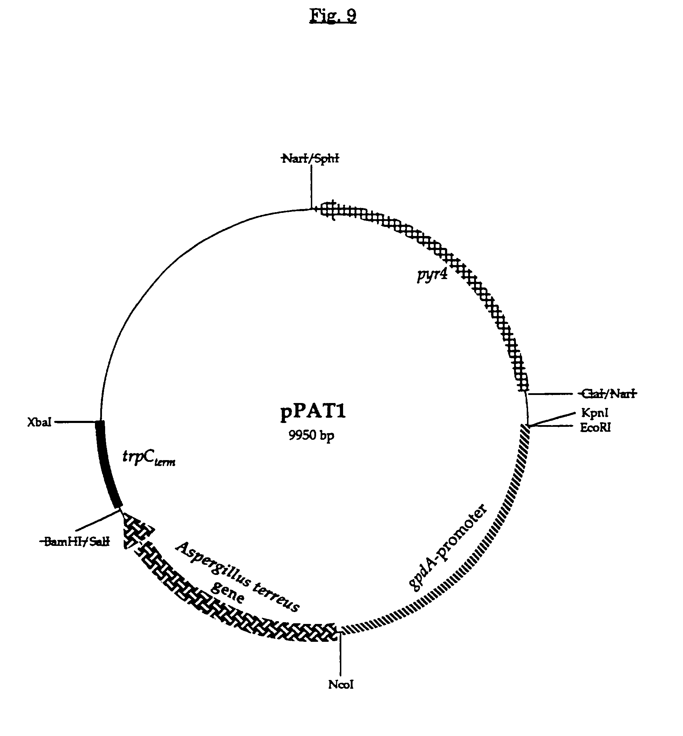

The *A. nidulans* glyceraldehyd-3-phosphate dehydrogenase *(gpdA)* promoter was isolated as a ~2.3 kb EcoRI/NcoI fragment from plasmid pAN52-1 [Punt et al. (1987), Gene 56: 117-124], cloned into pUC19-NcoI (pUC19 having a SmaI-site replaced by a NcoI-site), reisolated as EcoRI/ BamHI fragment and cloned into pBS with the *trpC* terminator as described above. The obtained cassette was named pGPDN. The *A*. *terreus* gene was isolatet as a NcoI/EcoRI fragment, where the EcoRI site was filled in to create blunt ends. Plasmid pGPDN was cut with BamHI and NcoI. The BamHI site was filled in to create blunt ends. The NcoI/EcoRI(blunt) fragment of the A. *terreus* gene was cloned between the *gpdA* promoter and *trpC* terminator. The expression cassette was isolated as KpnI/XbaI fragment and cloned into pUC19-*pyr4* resulting in plasmid pPAT1 (see Figure 9; for explanation of abreviations see legend to Figure 8).

### Expression of the fusion protein in Aspergillus niger

### A) Transformation

The plasmid pFPAN1 was used to transform *A*. *niger* by using the transformation protocol as described by Ballance et al. [(1983), Biochem. Biophys. Res. Commun 112, 284-289] with some modifications:
- YPD medium (1 % yeast extract, 2% peptone, 2 % dextrose) was inoculated with 10⁶ spores per ml and grown for 24 hours at 30° C and 250 rpm
- cells were harvested using Wero-Lene N tissue (No. 8011.0600 Wernli AG Verbandstoffabrik, 4852 Rothrist, CH) and once washed with buffer (0.8 M KCl, 0.05 M CaCl₂, in 0.01 M succinate buffer; pH 5.5)
- for protoplast preparation only lysing enzymes (SIGMA L-2265, St. Louis, MO, USA) were used
- the cells were incubated for 90 min at 30° C and 100 rpm, and the protoplasts were separated by filtration (Wero-Lene N tissue)
- the protoplasts were once washed with STC (1 M sorbitol, 0.05 M CaCl₂, 0.01 M Tris/HCl pH 7.5) and resuspended in the same buffer
- 150 µl protoplasts (~10⁸/ml) were gently mixed with 10-15 µg plasmid DNA and incubated at room temperature (RT) for 25 min
- polyethylene glycol (60% PEG 4000, 50 mMCaCl₂,10 mM Tris/HCl pH 7.5) was added in three steps, 150 µl, 200 µl and 900µl, and the sample was further incubated at room temperature (RT) for 25 min
- 5 ml STC were added, centrifuged and the protoplasts were resuspended in 2.5 ml YGS (0.5% yeast extract, 2% glucose, 1.2 M sorbitol)
- the sample was incubated for 2 hours at 30° C (100 rpm) centrifuged and the protoplasts were resuspended in 1 ml 1.2 M sorbitol
- the transformed protoplasts were mixed with 20 ml minimal regeneration medium (0.7% yeast nitrogen base without amino acids, 2% glucose, 1 M sorbitol, 1.5% agar, 20 mM Tris/HCl pH 7.5 supplemented with 0.2 g arginine and 10 mg nicotinamide per liter)
- the plates were incubated at 30° C for 3-5 days

### B) Expression

Single transformants were isolated, purified and tested for overproduction of the fusion protein. 100 ml M25 medium (70g maltodextrin (Glucidex 17D, Sugro Basel, CH), 12.5g yeast extract, 25g casein-hydrolysate, 2g KH₂PO₄, 2g K₂SO₄, 0.5g MgSO₄·7H₂O, 0.03g ZnCl₂, 0.02g CaCl₂, 0.05g MnSO₄·4H₂O, 0.05g FeSO₄ per liter pH 5.6) were inoculated with 10⁶ spores per ml from transformants FPAN1#11, #13, #16, #E25, #E30 respectively #E31 and incubated for 5 days at 30° C and 270 rpm. Supernatant was collected and the activity determined. The fusion protein showed the highest activity with phytic acid as substrate at pH 2.5, whereas with 4-nitrophenyl phosphate as substrate it showed two activity optima at pH 2.5 and 5.0 (Table 1).

### C) Activity assay

a) Phytic acid
   A 1 ml enzyme reaction contained 0.5 ml dialyzed supernatant (diluted if necessary) and 5.4 mM phytic acid (SIGMA P-3168). The enzyme reactions were made in 0.2 M sodium acetate buffer pH 5.0, respectively 0.2 M glycine buffer pH 2.5. The samples were incubated for 15 min at 37° C. The reactions were stopped by adding 1 ml 15% TCA (trichloroacetic acid).
   For the colour reaction 0.1 ml of the stopped sample was diluted with 0.9 ml destilled water and mixed with 1 ml reagent solution (3 volumes 1 M H₂SO₄, 1 volume 2.5% (NH₄)₆Mo₇O₂₄, 1 volume 10% ascorbic acid). The samples were incubated for 20 min at 50° C and the blue colour was measured spetrophotometrically at 820 nm. Since the assay is based on the release of phosphate a phosphate standard curve, 11- 45 nmol per ml, was used to determine the activity of the samples.
b) 4-nitrophenyl phosphate
   A 1 ml enzyme reaction contained 100 µl dialyzed supernatant (diluted if necessary) and 1.7 mM 4-nitrophenyl phosphate (Merck, 6850, Darmstadt, BRD). The enzyme reactions were made in 0.2 M sodium acetate buffer pH 5.0, respectively 0.2 M glycine buffer pH 2.5. The samples were incubated for 15 min at 37° C. The reactions were stopped by adding 1 ml 15% TCA.
   For the determination of the enzyme activity the protocol described above was used.

**TABLE 1**

| | SUBSTRATE | | | |
|---|---|---|---|---|
| Transformant | * Phytic Acid | | * 4-Nitrophenyl phosphate | |
| | pH 5.0 | pH 2.5 | pH 5.0 | pH 2.5 |
| A. niger ¹⁾ | 0.2 | 1 | 1 | 2 |
| FPAN1 # 11 | 6 | 49 | 173 | 399 |
| FPAN1 # 13 | 2 | 21 | 60 | 228 |
| FPAN1 # 16 | 1 | 16 | 46 | 153 |
| FPAN1 # E25 | 3 | 26 | 74 | 228 |
| FPAN1 # E30 | 3 | 43 | 157 | 347 |
| FPAN1 # E31 | 3 | 39 | 154 | 271 |

| | | | | |
|---|---|---|---|---|
| * Units per ml: 1 unit =1 µmol phosphate released per min at 37° C | | | | |
| ¹⁾ not tranformed | | | | |

### Expression of the Aspergillus terreus 9A1 gene in Aspergillus niger

*A. niger* NW205 was transformed with plasmid pPAT1 as described above. Single transformants were isolated, purified and screened for overproduction of the *A. terreus* protein. 50 ml YPD medium were inoculated with 10⁶ spores per ml from transformants PAT1#3, #10, #11, #13 and #16 and incubated for 3 days at 30° C and 270 rpm. Supernatant was collected and the activity determined as described above except that the pH for the enzyme reactions were different. The enzyme showed its main activity at pH 5.5 with phytic acid as substrate and at pH 3.5 with 4-nitrophenyl phosphate as substrate (Table 2).

**TABLE 2**

| | SUBSTRATE | | | |
|---|---|---|---|---|
| Transformant | * Phytic Acid | | * 4-Nitrophenyl phosphate | |
| | pH 5.5 | pH 3.5 | pH 5.5 | pH 3.5 |
| A. niger¹) | 0 | 0 | 0 | 0.1 |
| PAT1#3 | 10 | 0 | 0.2 | 0.7 |
| PAT1#10 | 9 | 0 | 0.2 | 0.8 |
| PAT1 # 11 | 5 | 0 | 0.1 | 0.5 |
| PAT1 # 13 | 9 | 0 | 0.2 | 0.7 |
| PAT1 # 16 | 5 | 0 | 0.1 | 0.5 |

| | | | | |
|---|---|---|---|---|
| * Units per ml: 1 unit =1 µmol phosphate released per min at 37° C | | | | |
| 1) not transformed | | | | |

### Example 10

### Fermentation of Aspergillus niger NW 205 transformants

### A) Transformant FPAN1#11

Preculture medium [30 g maltodextrin (Glucidex 17D), 5 g yeast extract, 10 g casein-hydrolysate, 1 g KH₂PO₄, 0.5g MgSO₄·7H₂O, 3 g Tween 80 per liter; pH 5.5] was inoculated with 10⁶ spores per ml in a shake flask and incubated for 24 hours at 34° C and 250 rpm.

A 10 liter fermenter was inoculated with the pre-culture to a final dilution of the pre-culture of 1:100. The batch fermentation was run at 30° C with an automatically controlled dissolved oxygen concentration of minimum 25% (pO₂ ≥ 25%). The pH was kept at 3.0 by automatic titration with 5 M NaOH.

The medium used for the fermentation was: 35 g maltodextrin, 9.4 g yeast extract, 18.7 g casein-hydrolysate, 2 g KH₂PO₄, 0.5 g MgSO₄·7H₂O, 2 g K₂SO₄, 0.03 g ZnCl₂, 0.02 g CaCl₂, 0.05 g MnSO₄·4H₂O, 0.05 g FeSO₄ per liter; pH 5.6.

Enzyme activities reached after 3 days under these conditions were 35 units/ml respectively 16 units/ml at pH 2.5 respectively pH 5.0 with phytic acid as substrate and 295 units/ml respectively 90 units/ml at pH 2.5 respectively pH 5.0 and 4-nitrophenyl phosphate as substrate.

### B) Transformant PAT1#11

Preculture, inoculation of the fermenter and the fermentation medium were as described above, except that the pH was kept at 4.5 by automatic titration with 5 M NaOH.

Enzyme activities reached after 4 days under these conditions were 17.5 units/ml at pH 5.5 with phytic acid as substrate and 2 units/ml at pH 3.5 with 4-nitrophenyl phosphate as substrate.

### Example 11

### Isolation of PCR fragments of a phytase gene of Aspergillus terreus (CBS 220.95)

Two different primer pairs were used for PCR amplification of fragments using DNA of *Aspergillus terreus* [CBS 220.95]. The primers used are shown in the Table below.

| **Fragment amplified** | **Primers** | **Oligonucleotide sequences (5' to 3')** |
|---|---|---|
| 8 plus 9 | 8 | ATGGA(C/T)ATGTG(C/T)TC(N)TT(C/T)GA [SEQ ID NO:8] |
| about | | Amino acids 254-259: MDMCSF |
| 150 bp | 9 | TT(A/G)CC(A/G)GC(A/G)CC(G/A)TG(N)CC(A/G)TA [SEQ ID NO:9] |
| | | Amino acids 296-301: YGHGAG |
| 10 plus 11 | 10 | TA(C/T)GC(N)GA(C/T)TT(C/T)TC(N)CA(C/T)GA [SEQ ID NO:10] |
| about | | Amino acids 349-354: YADFSH |
| 250 bp | 11 | *CG(G*/*A)TC(G*/*A)TT(N)AC(N)AG(N)AC(N)C* [SEQ ID NO:11] |
| | | Amino acids 416-422: RVLVNDR |

DNA sequences in bold show the sense primer and in italics the antisense primer. The primers correspond to the indicated part of the coding sequence of the *Aspergillus niger* gene. The combinations used are primers 8 plus 9 and 10 plus 11. The Taq-Start antibody kit from Clontech (Palo Alto, CA, USA) was used according to the manufacturer's protocol. Primer concentrations for 8 plus 9 were 0.2 mM and for primers 10 plus 11 one mM. Touch-down PCR was used for amplification [Don, R.H. et al. (1991), Nucleic Acids Res. 19, 4008]. First the DNA was denatured for 3 min at 95°C. Then two cycles were done at each of the following annealing temperatures: 60°C, 59°C, 58°C, 57°C, 56°C, 55°C, 54°C, 53°C, 52°C and 51°C, with an annealing time of one min. each. Prior to annealing the incubation was heated to 95°C for one min and after annealing elongation was performed for 30 sec at 72°C. Cycles 21 to 35 were performed as follows: denaturation one min at 95°C, annealing one min at 50°C and elongation for 30 sec at 72°C.

Two different PCR fragments were obtained. The DNA sequences obtained and their comparison to relevant parts of the phytase gene of Aspergillus terreus 9A1 are shown in Figure 10 [relevant parts of the phytase gene of *Aspergillus terreus* 9A1 "9A1"(top lines) (1) and the PCR fragments of *Aspergillus terreus* CBS 220.95 "aterr21" (bottom lines). Panel A: Fragment obtained with primer pair 8 plus 9 (aterr21). Panel B: Fragment obtained with primer pair 10 plus 11 (aterr58). DNA sequences of *Aspergillus terreus* CBS 220.95 (top lines) are compared with those of *Aspergillus terreus* 9A1 (1) (bottom lines). Panel A: The bold gc sequence (bases 16 plus 17) in the aterr21 fragment could possibly be cg (DNA sequencing uncertainty). Panel B: The x at position 26 of the aterr58 PCR fragment could possibly represent any of the four nucleotides].

### Example 12

### Cross hybridizations under non-stringent and stringent washing conditions

Five µg's of genomic DNA of each strain listed in Table 3 were incubated with 4 units of *Hind*III or *Pst*I, respectively, per µg of DNA at 37°C for 4 hours. After digestion, the mixtures were extracted with phenol and DNAs were precipitated with ethanol. Samples were then analyzed on 0.8% agarose gels. DNAs were transferred to Nytran membranes (Schleicher & Schuell, Keene, NH, USA) using 0.4M NaOH containing 1M NaCl as transfer solution. Hybridizations were performed for 18 hours at 42°C. The hybridization solution contained 50% formamide, 1% SDS, 10% dextran sulphate, 4 x SSPE (1 x SSPE = 0.18M NaCl, 1 mM EDTA, 10 mM NaH₂PO₄, pH 7.4), 0.5% blotto (dried milk powder in H₂O) and 0.5 mg salmon sperm DNA per ml. The membranes were washed under non-stringent conditions using as last and most-stringent washing condition incubation for 30 min at room temperature in 0.1 x SSPE containing 0.1 % SDS. The probes (labelled at a specific activity of around 10⁹ dpm/µg DNA) used were the PCR fragments generated with primers 8 plus 9 (see Example 11) using genomic DNA of *Myceliophthora thermophila; **Mycelio. thermo.**, ; Aspergillus nidulans, **Asperg. nidul.**; Aspergillus fumigatus, **Asperg. fumig.**,; Aspergillus terreus* 9A1, ***Asperg. terreus* 9A1**. *Talaromyces thermophilus, **Talarom. thermo.*** The **MT2** genomic probe was obtained by random priming (according to the protocol given by Pharmacia, Uppsala, Sweden) and spans 1410 bp, from the BspEI site upstream of the N-terminus of the ***Mycelio. thermo.*** phytase gen to the PvuII site in the C-terminus (positions 2068 to 3478). The AT2 genomic probe was obtained by random priming and spans 1365 bp, from the ApaI site to the NdeI site of the ***Asperg. terreus* 9A1** phytase gene (positions 491 to 1856). The **AN2** DNA probe was obtained by random priming and spans the complete coding sequence (1404 bp) of the ***Asperg. niger*** gene (EP 420 358). Results are given in Table 3. ["*"except for weak signal corresponding to a non-specific 20kb fragment; In case of the very weak cross-hybridization signal at 20 kb seen with DNA from *Aspergillus niger* using the PCR fragment from *Talaromyces thermophilus* this signal is unspecific, since it differs significantly from the expected 10 kb *Hind*III fragment, containing the phytase gene; "**" signal due to only partical digest of DNA].

For cross-hybridizations with stringent washing conditions membranes were further washed for 30 min. at 65°C in 0.1 x SSPE containing 0.1% SDS. Results are shown in Table 4 [(1) only the 10.5-kb HindIII fragment is still detected, the 6.5-kb HIndIII fragment disappeared (see table 3)].

**Table 3**

| | PCR Probes | | | | | Genomic Probes | | DNA Probes |
|---|---|---|---|---|---|---|---|---|
| Source of DNA used for cross-hybrization | Band (kb) detected with Probe of *Asperg. fumig.* | Band (kb) detected with Probe of *Asperg. nidul.* | Band (kb) detected with Probe of *Asperg. terreus* 9A1 | Band (kb) detected with Probe of *Mycelio. thermo.* | Band (kb) detected with Probe of *Talarom. thermo.* | Band (kb) detected with genomic Probe MT2 of *Mycelio. thermo.* | Band (kb) detected with genomic Probe AT2 of *Asperg. terreus 9A*1 | Band (kb) detected with cDNA Probe AN2 of *Asperg. niger* (*control*) |
| *Acrophialophora levis* [*ATCC 48380*] | no | no | no | no | no | 8-kb | no | no |
| *Aspergillus niger [ATCC 9142] (control)* | no | no | no | no | no* | no | no | 10 kb *Hind*III |
| *Aspergillus terreus [CBS 220.95]* | no | no | 11-kb *Hind*III | no | no | no | 11-kb *Hind*III | no |
| *Aspergillus sojae [CBS 221.95]* | no | no | no | no | no* | no | 3.7-kb *Hind*III | no |
| *Calcarisposiella thermophila [ATCC 22718]* | no | no | 10.5-kb *Hind*III | no | no | 10.5-kb *Hind*III | 10.5-kb *Hind*III | no |
| *Chaetomium rectopilium* [*ATCC 22431*] | no | no | no | no | no | >20-kb** *Hind*III | >20-kb** *Hind*III | no |
| *Corynascus thermophilus [ATCC 22066]* | no | no | no | no | no | 10.5-kb *Hind*III | no | no |
| *Humicola sp. [ATCC 60849]* | no | no | no | no | no | 9.5-kb *Hind*III | no | no |
| *Mycelia sterilia [ATCC 20350]* | no | no | no | 6-kb *Hind*III | no | 6-kb *Hind*III | 6-kb *Hind*III | no |
| *Myrococcum thermophilum [ATCC 22112]* no | no | no | no | no | 4.8-kb *Hind*III | no | no | no |
| *Rhizomucor miehei [ATCC 22064]* | no | 3.8-kb *Hind*III | no | no | no | no | no | no |
| *Sporotrichum cellulophilum [ATCC 20494]* | no | no | no | 6-kb *Hind*III | no | 6-kb and 10.5-kb *Hind*III | 6-kb and 10.5-kb *Hind*III | no |
| | | | | 2.1/3.7-kb *Pst*I | | | | |
| *Sporotrichum thermophile [ATCC 22482]* | no | no | no | 6-kb *Hind*III | 6-kb *Hind*III | 6-kb *Hind*III | 6-kb *Hind*III | no |
| | | | | 2.1/3.7-kb *Pst*I | | | | |
| *Scytalidium indonesicum [ATCC 46858]* | no | no | no | no | no | 9-kb *Hind*III | no | no |
| *Aspergillus fumigatus [ATCC 34625]* | 2.3-kb *Hind*III | no | no | no | no | no | no | no |
| *Aspergillus nidulans [DSM 9743]* | no | 9.5-kb *Hind*III | no | no | no | no | 9.5-kb *Hind*III | no |
| *Aspergillus terreus 9A1 [DSM 9076]* | no | no | 10.5-kb *Hind*III | no | 6.5-kb *Hind*III | 10.5-kb *Hind*III | 10.5-kb *Hind*III | no |
| *Myceliophthora thermophila* [*ATCC 48102*] | no | no | no | 6.5-kb *Hind*III | no | 6.5-kb *Hind*III | 6.5-kb *Hind*III | no |
| *Talaromyces thermophilus [ATCC 20186]* | no | no | no | no | 9.5-kb *Hind*III | no | no | no |

**Table 4**

| Source of DNA used for cross-hybriziation | Probe *Asperg. fumig.* | Probe *Asperg. nidul.* | Probe *Asperg. terreus* 9A1 | Probe *Mycelio. thermo.* | Probe *Talarom. thermo.* | Genomi c Probe of MT2 *Mycelio. thermo.* | Genomic Probe of AT2 *Asperg. terreus 9A1* | DNA Probe of AN2 *Asperg. niger (control)* |
|---|---|---|---|---|---|---|---|---|
| *Acrophiolophora levis* | | | | | | yes | | |
| *Aspergillus niger (control)* | | | | | | | | yes |
| *Aspergillus terreus* (CBS 116.46) | | | yes | | | | yes | |
| *Calcarisporiella thermophila* | | | yes | | | | yes | |
| *Chaetomium rectopilium* | | | | | | yes | | |
| *Corynascus thermophilus* | | | | | | yes | | |
| *Sporotrichum cellulophilum* | | | | yes | | yes | yes⁽¹⁾ | |
| *Sporotrichum thermophile* | | | | yes | | yes | | |
| *Aspergillus fumigatus* | yes | | | | | | | |
| *Aspergillusd nidulans* | | yes | | | | | | |
| *Aspergillus terreus 9A1* | | | yes | | | | yes | |
| *Mycelia sterilia* | | | | | | yes | | |
| *Myceliophthora thermophila* | | | | yes | | yes | | |
| *Talaromyces thermophilus* | | | | | yes | | | |

## Claims

1. A DNA sequence coding for a polypeptide having a heat tolerant phytase activity **with an activity maximum at around ph 5.5** and which DNA sequence is derived from a fungus selected from the group consisting of Aspergillus terreus, Myceliophtora thermophila, Aspergillus fumigatus, Aspergillus nidulans and Talaromyces thermophilus which DNA sequence is selected from the following:
(a1) the DNA sequence of Figure 1 [SEQ ID NO:1] or its complementary strand;
(a2) the DNA sequence of Figure 2 [SEQ ID NO:3] or its complementary strand;
(a3) a DNA sequence comprising one of the DNA sequences of Figures 4 [SEQ ID NO:5], 5 [SEQ ID NO:7], 6 [SEQ ID NO:9] or 10 [SEQ ID NO:13] and/or [SEQ ID NO:14] or its complementary strand;
(b) a DNA sequence which hybridizes under standard conditions with sequences defined under (a1-a3);
(c) a DNA sequence which, because of the degeneracy of the genetic code, does not hybridize with sequences of (a1-a3) of (b), but which codes for polypeptides having exactly the same amino acid sequences as the polypeptides encoded by these DNA sequences; and
(d) a DNA sequence which is a fragment of the DNA sequences specified in (a1-a3), (b) or (c).

2. A DNA sequence according to claim 1 which DNA sequence is selected from a DNA sequence comprising the DNA sequence of Figure 4 [SEQ ID NO:5] isolatable from Talaromyces thermophilus, of Figure 5 [SEQ ID NO:7] isolatable from Aspergillus fumigatus, of Figure 6 [SEQ ID NO:9] isolatable from Aspergillus nidulans or of Figure 10 [SEQ ID NO:13] and/or [SEQ ID NO:14] isolatable from Aspergillus terreus (CBS 220.95) or which DNA sequence is a degenerate variant or equivalent thereof.

3. A DNA sequence coding for a chimeric construct having phytase activity which chimeric construct comprises a fragment of a DNA sequence as claimed in any one of claims 1 or 2.

4. A DNA sequence coding for a chimeric construct as defined in claim 35 which chimeric construct consists at its N-terminal end of a fragment of the Aspergillus niger phytase fused at its C-terminal end to a fragment of the Aspergillus terreus phytase.

5. A DNA sequence as claimed in claim 4 with the specific nucleotide sequence as shown in Figure 7 [SEQ ID NO:11] and a degenerate variant or equivalent thereof.

6. A polypeptide encoded by a DNA sequence as claimed in any one of claims 1 to 58.

7. A vector comprising a DNA sequence as claimed in any one of claims 1 to 5.

8. A vector as claimed in claim 7 suitable for the expression of said DNA sequence in bacteria or a fungal or a yeast host.

9. Bacteria or a fungal or yeast host transformed by a DNA sequence as claimed in any one of claim 1 to 5 or a vector as claimed in claim 7 or 8

10. A composit food or feed comprising one or more polypeptides as defined in claim 69.

11. A process for the preparation of a polypeptide as claimed in claim 6 **characterized in that** transformed bacteria or host cell as claimed in claim 9 is cultured under suitable conditions and the polypeptide is recovered therefrom.

12. A polypeptide when produced by a process as claimed in claim 11.

13. A process for the preparation of a composit feed or food wherein the components of the composition are mixed with one or more polypeptides as defined in claim 69.

14. A process for the reduction of levels of phytate in anima I manure **characterized in that** an animal is fed a composit feed as defined in claim 10 in an amount effective in converting phytate contained in the feedstuff to inositol and inorganic phosphate.

15. Use of a polypeptide according to claim 6 for the conversion of phytate to inositol phosphates, inositol and inorganic phosphate

## Patentansprüche

1. DNA-Sequenz, die für ein Polypeptid codiert, welches eine wärmeverträgliche Phytaseaktivität mit einem Aktivitätsmaximum bei einem pH von rund 5,5 hat, und wobei die DNA-Sequenz von einem Fungus stammt, ausgewählt aus der Gruppe, bestehend aus Aspergillus terreus, Myceliophtora thermophila, Aspergillus fumigatus, Aspergillus nidulans und Talaromyces thermophilus, wobei die DNA-Sequenz aus folgendem ausgewählt ist:
(a1) der DNA-Sequenz von Figur 1 [SEQ ID Nr. 1] oder ihrem komplementären Strang;
(a2) der DNA-Sequenz von Figur 2 [SEQ ID Nr. 3] oder ihrem komplementären Strang; (a3) einer DNA-Sequenz, umfassend eine der DNA-Sequenzen der Figuren 4 [SEQ ID Nr. 5], 5 [SEQ ID NO.7], [SEQ ID NO.9 oder10 [SEQ 10 NO.13]) und/oder [SEQ ID NO.14] oder Ihrem komplementären Strang: (b) einer DNA-Sequenz, die unter Standardbedingungen mit Sequenzen, die unter (a1 bis a3) definiert sind, hybridisiert; (c) einer DNA-Sequenz, die aufgrund der Degeneration des genetischen Codes nicht mit den Sequenzen, die unter (a1 bis a3) definiert sind, hyubridisiert, aber welche Polypeptide kodieren, die genau die gleiche Aminosäuresequenz aufweisen, wie die durch diese DNA-Sequenzen codierten Polypeptide. (d) einer DNA-Sequenz, die ein Fragment der DNA-Sequenzen ist, spezifiziert in (a1 bis a3), (b)

2. DNA-Sequenz nach Anspruch 1, wobei die DNA-Sequenz aus einer DNA-Sequenz ausgewählt ist, umfassend die DNA-Sequenz von Figur 4 [SEQ ID Nr. 5], isolierbar aus Talaromyces thermophilus, von Figur 5 [SEQ ID Nr. 7], isolierbar aus Aspergillus fumigatus, von Figur 6 [SEQ ID Nr. 9], isolierbar aus Aspergillus nidulans, oder von Figur 10 [SEQ ID Nr. 13 und/oder SEQ ID Nr. 14], isolierbar aus Aspergillus terreus (CBS 220.95), oder wobei die DNA-Sequenz eine degenerierte Variante oder ein Äquivalent davon ist.

3. DNA-Sequenz, die für ein chimäres Konstrukt mit Phytaseaktivität codiert, wobei das chimäre Konstrukt ein Fragment einer DNA-Sequenz nach einem der Ansprüche 1 oder 2 umfaßt.

4. DNA-Sequenz, die für ein chimäres Konstrukt nach Anspruch 3 codiert, wobei das chimäre Konstrukt an seinem N-terminalen Ende aus einem Fragment der Aspergillus niger-Phytase besteht, die an ihrem C-terminalen Ende an ein Fragment der Aspergillus terreus-Phytase fusioniert ist.

5. DNA-Sequenz nach Anspruch 4 mit der spezifischen Nucleotidsequenz, wie in Figur 7 [SEQ ID Nr. 11] gezeigt, und eine degenerierte Variante oder ein Äquivalent davon.

6. Polypeptid, das durch eine DNA-Sequenz nach einem der Ansprüche 1 bis 5 codiert wird.

7. Vektor, umfassend eine DNA-Sequenz nach einem der Ansprüche 1 bis 5.

8. Vektor nach Anspruch 7, der zur Expression der DNA-Sequenz in Bakterien oder einem Fungus- oder Hefewirt geeignet ist.

9. Bakterien oder ein Fungus- oder Hefewirt, transformiert durch eine DNA-Sequenz nach einem der Ansprüche 1 bis 5 oder einen Vektor nach Anspruch 7 oder 8.

10. Mischnahrungsmittel oder -futter, umfassend ein oder mehrer Polypeptide nach Anspruch 6.

11. Verfahren zur Herstellung eines Polypeptids nach Anspruch 6, **dadurch gekennzeichnet, daß** die transformierten Bakterien oder die transformierte Wirtszelle nach Anspruch 9 unter geeigneten Bedingungen kultiviert werden und das Polypeptid daraus rückgewonnen wird.

12. Polypeptid, hergestellt durch ein Verfahren nach Anspruch 11.

13. Verfahren zur Herstellung eines Mischfutters oder -nahrungsmittels, wobei die Komponenten der Zusammensetzung mit einem oder mehreren Polypeptiden nach Anspruch 6 gemischt werden.

14. Verfahren zur Reduktion des Phytatgehalts in Tiermist, **dadurch gekennzeichnet, daß** einem Tier ein Mischfutter nach Anspruch 10 in einer Menge gefüttert wird, die bei der Umwandlung von Phytat, das in dem Nahrungsmittel enthalten ist, zu Inositol und anorganischem Phosphat wirksam ist.

15. Verwendung eines Polypeptids nach Anspruch 6 zur Umwandlung von Phytat zu Inositolphosphaten, Inositol und anorganischem Phosphat.

## Revendications

1. Séquence d'ADN codant pour un polypeptide ayant une activité de phytase thermotolérante, avec une activité maximale aux environs de pH 5,5, ladite séquence d'ADN dérivant d'un champignon choisi dans le groupe consistant en Aspergillus terreus, Myceliophthora thermophila, Aspergillus fumigatus, Aspergillus nidulans et Talaromyces thermophilus, ladite séquence d'ADN étant choisie parmi les suivantes :
(a1) la séquence d'ADN de la Figure 1 [SEQ ID NO:1] ou son brin complémentaire ;
(a2) la séquence d'ADN de la Figure 2 [SEQ ID NO:3] ou son brin complémentaire ;
(a3) une séquence d'ADN comprenant l'une des séquences d'ADN de la Figure 4 [SEQ ID NO:5], 5 [SEQ ID NO:7], 6 [SEQ ID NO:9] ou 10 [SEQ ID NO:13] et/ou [SEQ ID NO:14] ou son brin complémentaire ;
(b) une séquence d'ADN qui s'hybride dans des conditions standard aux séquences définies en (a1-a3) ;
(c) une séquence d'ADN qui, en raison de la dégénérescence du code génétique, ne s'hybride pas aux séquences (a1-a3) de (b), mais qui code pour des polypeptides ayant exactement les mêmes séquences d'acides aminés que les polypeptides codés par ces séquences d'ADN ; et
(d) une séquence d'ADN qui est un fragment des séquences d'ADN spécifiées en (a1-a3), (b) ou (c).

2. Séquence d'ADN selon la revendication 1, ladite séquence d'ADN étant choisie parmi une séquence d'ADN comprenant la séquence d'ADN de la Figure 4 [SEQ ID NO:5] pouvant être isolée de Talaromyces thermophilus, de la Figure 5 [SEQ ID NO:7] pouvant être isolée d'Aspergillus fumigatus, de la Figure 6 [SEQ ID NO:9] pouvant être isolée d'Aspergillus nidulans ou de la Figure 10 [SEQ ID NO:13] et/ou [SEQ ID NO:14] pouvant être isolée d'Aspergillus terreus (CBS 220.95), ou encore ladite séquence d'ADN en étant un variant dégénéré ou un équivalent.

3. Séquence d'ADN codant pour une construction chimère ayant une activité de phytase, ladite construction chimère comprenant un fragment d'une séquence d'ADN selon l'une quelconque des revendications 1 ou 2.

4. Séquence d'ADN codant pour une construction chimère selon la revendication 3, laquelle construction chimère consiste, en son extrémité N-terminale, en un fragment de la phytase d'Aspergillus niger, fusionné au niveau de son site C-terminal à un fragment de la phytase d'Aspergillus terreus.

5. Séquence d'ADN selon la revendication 4, ayant la séquence nucléotidique spécifique telle que présentée sur la Figure 7 [SEQ ID NO:11], et un variant dégénéré ou un équivalent de cette dernière.

6. Polypeptide codé par une séquence d'ADN selon l'une quelconque des revendications 1 à 5.

7. Vecteur comprenant une séquence d'ADN selon l'une quelconque des revendications 1 à 5.

8. Vecteur selon la revendication 7, convenant à l'expression de ladite séquence d'ADN dans des bactéries ou dans un hôte fongique ou de levure.

9. Bactérie, ou hôte fongique ou de levure, transformé par une séquence d'ADN selon l'une quelconque des revendications 1 à 5 ou un vecteur selon la revendication 7 ou 8.

10. Aliment composé pour l'alimentation humaine ou animale, comprenant un ou plusieurs polypeptides selon la revendication 6.

11. Procédé de préparation d'un polypeptide selon la revendication 6, **caractérisé en ce que** la bactérie ou la cellule hôte transformée selon la revendication 9 est cultivée dans des conditions appropriées, et le polypeptide en est récupéré.

12. Polypeptide, quand il est produit par un procédé selon la revendication 11.

13. Procédé de préparation d'un aliment composé pour l'alimentation humaine ou animale, dans lequel les composants de la composition sont mélangés à un ou plusieurs polypeptides selon la revendication 6.

14. Procédé de réduction des niveaux de phytate dans un engrais animal, **caractérisé en ce qu'**on alimente un animal avec un aliment composé pour l'alimentation animale selon la revendication 10 en une quantité suffisante pour convertir le phytate contenu dans l'aliment en inositol et en phosphate inorganique.

15. Utilisation d'un polypeptide selon la revendication 6 pour convertir le phytate en phosphates d'inositol, en inositol et en phosphate inorganique.
